# EUROPÄISCHE PATENTSCHRIFT

(11) **EP 2 706 056 B1**
(45) Veröffentlichungstag und Bekanntmachung des Hinweises auf die Patenterteilung: **20.07.2016**
(21) Anmeldenummer: 13180047.6
(22) Anmeldetag: 12.08.2013
(51) Int. Cl.: C07D 211/74

(54) **Verfahren zur Herstellung und Aufarbeitung eines Triacetonamin-haltigen Reaktionsgemisches**
Method for the preparation and treatment of a reaction mixture containing triacetonamine
Procédé de fabrication et de traitement d'un mélange réactif contenant de la triacétonamine

(30) Priorität: 07.09.2012 DE 102012215900
(43) Veröffentlichungstag der Anmeldung: 12.03.2014
(73) Patentinhaber: Evonik Degussa GmbH, 45128 Essen (DE)
(72) Erfinder: Niemeyer, Jochen, 48153 Münster (DE); Neumann, Manfred, 45770 Marl (DE); Brehme, Volker, 48301 Nottuln (DE); Michel, Mirko, 44227 Dortmund (DE); Schwarz, Christoph, 45768 Marl (DE)

(56) Entgegenhaltungen:
- JP-A- 2003 206 277

## Beschreibung

Die vorliegende Erfindung betrifft ein Verfahren zur Herstellung von Triacetonamin, umfassend die Reaktion von Aceton und Ammoniak in Gegenwart eines Katalysators, und die daran anschließende Aufarbeitung des erhaltenen Reaktionsgemisches.

### Hintergrund der Erfindung

Triacetonamin (2,2,6,6-Tetramethyl-4-piperidinon; TAA) ist ein wichtiges chemisches Intermediat, welches zur Synthese zahlreicher Folgeprodukte eingesetzt wird. Wichtige Folgeprodukte sind dabei beispielsweise Lichtstabilisatoren (hindered amine light stabilizers [HALS]), Oxidationsmittel und Polymerisationsmoderatoren (z. B. Nitroxyl-Radikale).

Die Herstellung von Triacetonamin aus Aceton und Ammoniak ist in der Form verschiedener Verfahren dokumentiert. Dabei gliedern sich die Herstellungsverfahren zunächst grob in die direkte (einschrittige) Synthese von TAA aus den Edukten, beispielsweise beschrieben in DE2429937, US4536581, JP54088275 oder in Zeitschrift für Naturforschung 1976, 328-337 und 338-345, sowie die indirekte (zweistufige) Synthese über Acetonin (2,2,4,4,6-Pentamethyl-1,2,5,6-tetrahydropyrimidin), beispielsweise beschrieben in DE2429935 oder DE2429936, oder über Phoron (2,6-Dimethyl-2,5-heptadien-4-on), z.B. in DE2352127. Bei der zweistufigen TAA-Synthese über Acetonin wird dies zunächst ausgehend von Aceton und Ammoniak gebildet, und kann dann in einem anschließenden Schritt unter Abspaltung von einem Äquivalent Ammoniak weiter zu TAA reagieren. Im Falle des Syntheseverfahrens über Acetonin werden jedoch immer beide Spezies (TAA und Acetonin) gleichzeitig gebildet, die Acetoninbildung ist allerdings kinetisch gegenüber der TAA-Bildung stark bevorzugt. In der "einstufigen" TAA-Synthese wird das entstehende Acetonin lediglich nicht isoliert.

Im Stand der Technik wird von einer TAA-Synthese ausgehend von Aceton bzw. Aceton-Kondensationsprodukten gesprochen. Dies ist darin begründet, dass bei der Reaktion von Aceton und Ammoniak zunächst acyclische Additions- und Kondensationsprodukte (z. B. Diacetonalkohol [4-Hydroxy-4-methylpentan-2-on], Diacetonamin [4-Amino-4-methylpentan-2-on], Mesityloxid [4-Methylpent-3-en-2-on], Phoron etc.) gebildet werden. Diese Zwischenprodukte reagieren dann weiter zu TAA und können deshalb auch direkt als Edukte der Reaktion eingesetzt werden können, z. B. im Sinne eines prozessinternen Recyclestroms.

Die Herstellung von TAA ist grundsätzlich sowohl homogen katalysiert (meist durch Ammoniumsalze) als auch heterogen katalysiert (z. B. an sauren lonentauschern) möglich.

Die meisten Schriften aus dem Stand der Technik beziehen sich auf homogen katalysierte Reaktionen. Am häufigsten werden dabei Calciumchlorid (z.B. in Chemical Industries 2003, 89, 559-564; Zeitschrift für Naturforschung 1976, 328-337 und 338-345), Ammoniumchlorid (z.B. in JP2003-206277; JP2001-031651; JP04154762) und Hydrazinderivate (z.B. in JP54088275; JP54112873A) genannt. Bei Einsatz dieser Katalysatoren treten allerdings Probleme auf. So hat etwa der Einsatz von Calciumchlorid den Nachteil, dass eine sehr langsame Reaktion erfolgt. Im Falle des Ammoniumchlorids ist die Reaktionsgeschwindigkeit höher, das eingesetzte Chlorid zeigt allerdings eine hohe Korrosivität gegenüber Stahl. Hydrazinderivate zeigen demgegenüber eine sehr hohe Toxizität.

Die Herstellung ist sowohl im Batch-Verfahren (siehe die oben genannten Dokumente) als auch im Konti-Verfahren (z. B. in JP02204480; JP02145571) beschrieben.

Im Allgemeinen wird TAA in einer Matrix hergestellt, bei der das Aceton in großem Überschuss vorliegt und sowohl als Reaktionspartner als auch als Lösungsmittel dient. Daher ergibt sich am Ende der Reaktion ein Rohprodukt, welches neben TAA einen großen Anteil an Aceton, sowie nicht umgesetzten Ammoniak, durch die Kondensation gebildetes Wasser und bei homogenkatalytischen Verfahren den Katalysator enthält. Darüber hinaus sind weitere Nebenkomponenten enthalten, wie z.B. acyclische Kondensationsprodukte (z.B. Diacetonalkohol, Diacetonamin, Mesityloxid, Phoron etc.), cyclische Kondensationsprodukte (z.B. Acetonin, TMDH-Pyridin [2,2,4,6-Tetramethyl-2,3-dihydropyridin]) oder höhermolekulare Kondensationsprodukte ("Hochsieder").

Die gängigsten Verfahren zur Isolation von reinem TAA aus den genannten Mischungen sind insbesondere die destillative Aufarbeitung und die Kristallisation.

Die destillative Aufarbeitung kann sowohl im Batch-Verfahren (z.B. in JP2003-206277; JP2001-031651; JP04154763; DE 2429937) als auch im Konti-Verfahren (z.B. in JP2003-160561) durchgeführt werden. Es ist bekannt, dass bei homogenkatalysierten Verfahren, die z.B. unter Verwendung von Ammoniumsalzen durchgeführt werden, der saure Katalysator vor der Destillation durch Zugabe von Base deaktiviert werden muss, da sonst bei der Destillation eine Zersetzung des Produktes stattfindet (z.B. in JP04154763).

Im Falle der Kristallisation erfolgt in der Regel eine Zugabe von Wasser zu einer konzentrierten Lösung von TAA in einem unpolaren Lösungsmittel (z. B. in Kohlenwasserstoffen), Ausfällen des TAA-Hydrats, gegebenenfalls Waschen desselben mit einem weiteren Lösungsmittel. Dieser Prozessschritt erfolgt meist in Kombination mit einer vorherigen destillativen Abtrennung der Leichtsieder und ersetzt somit lediglich die Reindestillation (z. B. in JP04154763).

Die im Stand der Technik beschriebenen Verfahren zur homogen katalysierten Herstellung von TAA gehen hauptsächlich auf die Reaktion, die Deaktivierung des Katalysators sowie eine anschließende Isolierung des TAA durch eine Batch-Destillation ein (z.B. JP2001-031651, JP04154763, US4536581, JP 05140104). Diese beschriebenen Verfahren sind für die industrielle Herstellung wenig geeignet, da sie entweder problematisch bei der Durchführung sind oder sich Probleme bei der Aufarbeitung ergeben.
So führt die Synthese unter Verwendung von anorganischen Salzen als Katalysatoren dazu, dass spezielle und kostenintensive Stähle für den Anlagenbau verwendet werden müssen, um Korrosionsprobleme zu vermeiden. Dies ist im Besonderen bei Verwendung halogenid-haltiger Katalysatoren der Fall (so z.B. bei den im Stand der Technik am häufigsten genannten Katalysatoren, Ammoniumchlorid und Calciumchlorid), welche die Verwendung vieler Stähle und Edelstähle nicht erlauben.
Darüber hinaus ergibt sich bei allen homogen gelösten anorganischen Katalysatoren das Problem, dass sich diese bei einer destillativen Aufarbeitung des Rohproduktes im Destillationssumpf akkumulieren. Diese Akkumulation führt insbesondere im Hinblick auf eine industrielle Herstellung zu technischen Problemen, da sich Ablagerungen in der Destillationsblase oder am Wärmetauscher bilden können. Einige Dokumente des Standes der Technik (z.B. JP2001-031651, JP04154763, US4536581, JP05140104) beschreiben zwar die Zugabe einer Base und die anschließende Abtrennung einer wässrigen Phase, jedoch dient dies lediglich der oben beschriebenen notwendigen Deaktivierung des Katalysators (z.B. Zugabe von NaOH bei Verwendung von NH₄Cl als Katalysator und Bildung von NaCl, Wasser und Ammoniak). Nach der Abtrennung der wässrigen Phase befindet sich weiter ein Teil des deaktivierten Katalysators in der organischen Phase und wird bei einer Destillation im Sumpf akkumuliert.

Eine vollständige Entfernung des deaktivierten Katalysators ist zwar technisch möglich, jedoch sind die bekannten Verfahren zur Entfernung des Salzanteils durch Extraktion ihrerseits ebenfalls problematisch, da sie den Einsatz einer großen Menge an Hilfsstoffen erfordern, was zu einer große Menge an problematischen Abwässern führt. So führt das mehrmalige, sequentielle Versetzen des Rohproduktes mit einer Base, jeweils gefolgt von einer Phasentrennung (Kreuzstromextraktion; siehe z.B. K. Sattler, Thermische Trennverfahren, 3. Auflage, 2001, Wiley-VCH Weinheim, S. 542ff.) zwar zu einer vollständigen Entsalzung, jedoch nur unter Verwendung einer großen Menge an Base und deren Zugabe in fester Form.
Die alternative Möglichkeit einer Gegenstromextraktion (einmalige Zugabe von frischen Extraktionsmittel im letzten Extraktionsschritt und Rückführung des Extraktes in den vorhergehenden Extraktionsschritt) beschreibt JP2003-206277. In JP2003-206277 wird eine zweistufige Gegenstromextraktion beschrieben, sie kann aber auch mit größerer Stufenzahl betrieben werden. Jedoch weist auch das in JP2003-206277 beschriebene Verfahren die oben genannten Nachteile auf. So wird eine hinreichende Entsalzung nur durch Verwendung einer großen Menge an Base erreicht, was zur Bildung eines großen Abfallstromes führt.

Aufgabe der vorliegenden Erfindung ist somit die Bereitstellung eines verbesserten Verfahrens zur industriellen Herstellung von Triacetonamin. Dieses Verfahren soll dabei die oben genannten Probleme beheben. Es soll somit insbesondere die Menge der anfallenden Abfallstoffe verringern. Es soll unter Verwendung eines kostengünstigen und untoxischen Katalysators, der auch in einer kontinuierlichen Prozessführung effizient eingesetzt werden kann, ablaufen. Schließlich soll das verbesserte Verfahren auch eine vereinfachte und effiziente Aufarbeitung des Triacetonamins ermöglichen.

### Kurzbeschreibung der Erfindung

Es wurde nun überraschenderweise gefunden, dass die oben genannte Aufgabe durch ein Verfahren gemäß der vorliegenden Erfindung gelöst wird. Demgemäß ist ein erster Gegenstand der vorliegenden Erfindung
1. ein Verfahren zur Herstellung von Triacetonamin, umfassend die Reaktionsschritte
   a) Umsetzung eines Acetonäquivalents mit einer ersten Base, ausgewählt aus Ammoniak, in Gegenwart eines Katalysators, wobei ein Austragsgemisch erhalten wird, welches Aceton enthält;
   b) Deaktivierung des Austragsgemisches aus Schritt a) durch Zugabe von mindestens 1 molaren Äquivalent, bezogen auf die Menge des in Schritt a) des Verfahrens eingesetzten Katalysators, einer weiteren Base, wobei ein Triacetonamin-Rohprodukt erhalten wird, welches Aceton enthält;
   c) Entfernung von Aceton aus dem Triacetonamin-Rohprodukt aus Schritt b), wodurch ein acetonabgereichertes Triacetonamin-Rohprodukt erhalten wird, welches einen Acetongehalt von unter 40% (Massenprozent) aufweist;
   d) Extraktion des acetonabgereicherten Triacetonamin-Rohprodukts aus Schritt c) durch Zusatz eines Hilfsstoffs, welcher eine Base ist, wobei ein extrahiertes Triacetonamin-Rohprodukt erhalten wird.
2. In einer weiteren Ausführungsform der Erfindung ist das Verfahren gemäß Gegenstand 1 dadurch gekennzeichnet, dass in einem weiteren Schritt e) das extrahierte Triacetonamin-Rohprodukt aus Schritt d) zu Triacetonamin gereinigt wird.
   Das erfindungsgemäße Verfahren hat den Vorteil, dass der in Schritt a) eingesetzte und in Schritt b) deaktivierte Katalysator vor der Reinigung des extrahierten Triacetonamin-Rohprodukts in Schritt e), welche beispielsweise durch Destillation erfolgen kann, entfernt wird. Dies verhindert eine Akkumulation des deaktivierten Katalysators im Destillationssumpf. Dabei erlaubt es das erfindungsgemäße Verfahren, den Anteil des deaktivierten Katalysators bis unter die Nachweisgrenze zu reduzieren, und dabei eine im Vergleich zum Stand der Technik deutlich geringere Menge an Hilfsstoffen zu verwenden, wodurch auch die Menge der entstehenden Abfallströme gering gehalten wird.
3. In einer weiteren Ausführungsform der Erfindung ist das Verfahren gemäß einem oder mehreren der Gegenstände 1 bis 2 dadurch gekennzeichnet, dass das Acetonäquivalent eine oder mehrere der Verbindungen aus der Gruppe bestehend aus Aceton, Mesityloxid, Diacetonalkohol, Phoron, Diacetonamin, Acetonin ist.
4. In einer weiteren Ausführungsform der Erfindung ist das Verfahren gemäß einem oder mehreren der Gegenstände 1 bis 3 dadurch gekennzeichnet, dass das Acetonäquivalent Aceton ist.
5. In einer weiteren Ausführungsform der Erfindung ist das Verfahren gemäß einem oder mehreren der Gegenstände 1 bis 4 dadurch gekennzeichnet, dass die Entfernung von Aceton aus dem Triacetonamin-Rohprodukt in Schritt c) destillativ erfolgt.
6. In einer weiteren Ausführungsform der Erfindung ist das Verfahren gemäß einem oder mehreren der Gegenstände 1 bis 5 dadurch gekennzeichnet, dass die weitere Base in Schritt b) ausgewählt ist aus der Gruppe bestehend aus einem Alkalimetallhydroxid, einem Erdalkalimetallhydroxid, einem Alkalimetallcarbonat, einem Erdalkalimetallcarbonat.
7. In einer weiteren Ausführungsform der Erfindung ist das Verfahren gemäß einem oder mehreren der Gegenstände 1 bis 6 dadurch gekennzeichnet, dass als weitere Base in Schritt b) Natriumhydroxid oder Kaliumhydroxid eingesetzt wird.
8. In einer weiteren Ausführungsform der Erfindung ist das Verfahren gemäß einem oder mehreren der Gegenstände 1 bis 7 dadurch gekennzeichnet, dass als weitere Base in Schritt b) Natriumhydroxid eingesetzt wird.
9. In einer weiteren Ausführungsform der Erfindung ist das Verfahren gemäß einem oder mehreren der Gegenstände 1 bis 8 dadurch gekennzeichnet, dass als weitere Base in Schritt b) eine wässrige Lösung von Natriumhydroxid eingesetzt wird.
10. In einer weiteren Ausführungsform der Erfindung ist das Verfahren gemäß einem oder mehreren der Gegenstände 1 bis 9 dadurch gekennzeichnet, dass als weitere Base in Schritt b) eine wässrige Lösung von Natriumhydroxid, enthaltend 40-60% (Massenprozent) NaOH, eingesetzt wird.
11. In einer weiteren Ausführungsform der Erfindung ist das Verfahren gemäß einem oder mehreren der Gegenstände 1 bis 10 dadurch gekennzeichnet, dass als weitere Base in Schritt b) eine wässrige Lösung von Natriumhydroxid, enthaltend 45-55% (Massenprozent) NaOH, eingesetzt wird.
12. In einer weiteren Ausführungsform der Erfindung ist das Verfahren gemäß einem oder mehreren der Gegenstände 1 bis 7 dadurch gekennzeichnet, dass als weitere Base in Schritt b) Kaliumhydroxid eingesetzt wird.
13. In einer weiteren Ausführungsform der Erfindung ist das Verfahren gemäß einem oder mehreren der Gegenstände 1 bis 7 und 12 dadurch gekennzeichnet, dass als weitere Base in Schritt b) eine wässrige Lösung von Kaliumhydroxid eingesetzt wird.
14. In einer weiteren Ausführungsform der Erfindung ist das Verfahren gemäß einem oder mehreren der Gegenstände 1 bis 7 und 12 bis 13 dadurch gekennzeichnet, dass als weitere Base in Schritt b) eine wässrige Lösung von Kaliumhydroxid, enthaltend 40-60% (Massenprozent) KOH, eingesetzt wird.
15. In einer weiteren Ausführungsform der Erfindung ist das Verfahren gemäß einem oder mehreren der Gegenstände 1 bis 7 und 10 bis 14 dadurch gekennzeichnet, dass als weitere Base in Schritt b) eine wässrige Lösung von Kaliumhydroxid, enthaltend 45-55% (Massenprozent) KOH, eingesetzt wird.
16. In einer weiteren Ausführungsform der Erfindung ist das Verfahren gemäß einem oder mehreren der Gegenstände 1 bis 15 dadurch gekennzeichnet, dass die Menge an in Schritt b) zugegebener weiterer Base im Bereich von 1.0 bis 2.0 molaren Äquivalenten, bezogen auf die Menge des in Schritt a) eingesetzten Katalysators, liegt.
17. In einer weiteren Ausführungsform der Erfindung ist das Verfahren gemäß einem oder mehreren der Gegenstände 1 bis 16 dadurch gekennzeichnet, dass die Menge an in Schritt b) zugegebener weiterer Base im Bereich von 1.0 bis 1.4 molaren Äquivalenten, bezogen auf die Menge des in Schritt a) eingesetzten Katalysators, liegt.
18. In einer weiteren Ausführungsform der Erfindung ist das Verfahren gemäß einem oder mehreren der Gegenstände 1 bis 17 dadurch gekennzeichnet, dass die Menge an in Schritt b) zugegebener weiterer Base 1.2 molare Äquivalente, bezogen auf die Menge des in Schritt a) eingesetzten Katalysators, beträgt.
19. In einer weiteren Ausführungsform der Erfindung ist das Verfahren gemäß einem oder mehreren der Gegenstände 1 bis 18 dadurch gekennzeichnet, dass als Hilfsstoff in Schritt d) eine Base ausgewählt aus der Gruppe bestehend aus einem Alkalimetallhydroxid, einem Erdalkalimetallhydroxid, einem Alkalimetallcarbonat, einem Erdalkalimetallcarbonat eingesetzt wird.
20. In einer weiteren Ausführungsform der Erfindung ist das Verfahren gemäß einem oder mehreren der Gegenstände 1 bis 19 dadurch gekennzeichnet, dass als Hilfsstoff in Schritt d) eine Base ausgewählt aus der Gruppe bestehend aus Natriumhydroxid oder Kaliumhydroxid eingesetzt wird.
21. In einer weiteren Ausführungsform der Erfindung ist das Verfahren gemäß einem oder mehreren der Gegenstände 1 bis 20 dadurch gekennzeichnet, dass als Hilfsstoff in Schritt d) eine Base, nämlich Natriumhydroxid, eingesetzt wird.
22. In einer weiteren Ausführungsform der Erfindung ist das Verfahren gemäß einem oder mehreren der Gegenstände 1 bis 21 dadurch gekennzeichnet, dass als Hilfsstoff in Schritt d) eine Base, nämlich eine wässrige Lösung von Natriumhydroxid, eingesetzt wird.
23. In einer weiteren Ausführungsform der Erfindung ist das Verfahren gemäß einem oder mehreren der Gegenstände 1 bis 22 dadurch gekennzeichnet, dass als Hilfsstoff in Schritt d) eine Base, nämlich eine wässrige Lösung von Natriumhydroxid, enthaltend 40-60% (Massenprozent) NaOH, eingesetzt wird.
24. In einer weiteren Ausführungsform der Erfindung ist das Verfahren gemäß einem oder mehreren der Gegenstände 1 bis 23 dadurch gekennzeichnet, dass als Hilfsstoff in Schritt d) eine Base, nämlich eine wässrige Lösung von Natriumhydroxid, enthaltend 45-55% (Massenprozent) NaOH, eingesetzt wird.
25. In einer weiteren Ausführungsform der Erfindung ist das Verfahren gemäß einem oder mehreren der Gegenstände 1 bis 20 dadurch gekennzeichnet, dass als Hilfsstoff in Schritt d) eine Base, nämlich Kaliumhydroxid, eingesetzt wird.
26. In einer weiteren Ausführungsform der Erfindung ist das Verfahren gemäß einem oder mehreren der Gegenstände 1 bis 20 und 25 dadurch gekennzeichnet, dass als Hilfsstoff in Schritt d) eine Base, nämlich eine wässrige Lösung von Kaliumhydroxid, eingesetzt wird.
27. In einer weiteren Ausführungsform der Erfindung ist das Verfahren gemäß einem oder mehreren der Gegenstände 1 bis 20 und 25 bis 26 dadurch gekennzeichnet, dass als Hilfsstoff in Schritt d) eine Base, nämlich eine wässrige Lösung von Kaliumhydroxid, enthaltend 40-60% (Massenprozent) KOH, eingesetzt wird.
28. In einer weiteren Ausführungsform der Erfindung ist das Verfahren gemäß einem oder mehreren der Gegenstände 1 bis 20 und 25 bis 27 dadurch gekennzeichnet, dass als Base in Schritt d) eine wässrige Lösung von Kaliumhydroxid, enthaltend 45-55% (Massenprozent) KOH, eingesetzt wird.
29. In einer weiteren Ausführungsform der Erfindung ist das Verfahren gemäß einem oder mehreren der Gegenstände 1 bis 28 dadurch gekennzeichnet, dass die Menge an in Schritt d) als Hilfsstoff zugesetzter Base im Bereich von 1.0 bis 6.0 molaren Äquivalenten, bezogen auf die Menge des in Schritt a) eingesetzten Katalysators, liegt.
30. In einer weiteren Ausführungsform der Erfindung ist das Verfahren gemäß einem oder mehreren der Gegenstände 1 bis 29 dadurch gekennzeichnet, dass die Menge an in Schritt d) als Hilfsstoff zugesetzter Base im Bereich von 1.0 bis 4.0 molaren Äquivalenten, bezogen auf die Menge des in Schritt a) eingesetzten Katalysators, liegt.
31. In einer weiteren Ausführungsform der Erfindung ist das Verfahren gemäß einem oder mehreren der Gegenstände 1 bis 30 dadurch gekennzeichnet, dass die Menge an in Schritt d) als Hilfsstoff zugesetzter Base im Bereich von 1.0 bis 2.0 molaren Äquivalenten, bezogen auf die Menge des in Schritt a) eingesetzten Katalysators, liegt.
32. In einer weiteren Ausführungsform der Erfindung ist das Verfahren gemäß einem oder mehreren der Gegenstände 1 bis 31 dadurch gekennzeichnet, dass die Menge an in Schritt d) als Hilfsstoff zugesetzter Base im Bereich von 1.0 bis 1.4 molaren Äquivalenten, bezogen auf die Menge des in Schritt a) eingesetzten Katalysators, liegt.
33. In einer weiteren Ausführungsform der Erfindung ist das Verfahren gemäß einem oder mehreren der Gegenstände 1 bis 32 dadurch gekennzeichnet, dass die Menge an in Schritt d) als Hilfsstoff zugesetzter Base 1.2 molare Äquivalente, bezogen auf die Menge des in Schritt a) eingesetzten Katalysators, beträgt.
34. In einer weiteren Ausführungsform der Erfindung ist das Verfahren gemäß einem oder mehreren der Gegenstände 1 bis 33 dadurch gekennzeichnet, dass zusätzlich zwischen Schritt b) und Schritt c) eine Phasentrennung erfolgt.
35. In einer weiteren Ausführungsform der Erfindung ist das Verfahren gemäß einem oder mehreren der Gegenstände 1 bis 34 dadurch gekennzeichnet, dass zusätzlich zwischen Schritt b) und Schritt c) eine Phasentrennung erfolgt, und in den Schritten c) bis d) die organische Phase des Triacetonamin-Rohproduktes verwendet wird.
36. In einer weiteren Ausführungsform der Erfindung ist das Verfahren gemäß einem oder mehreren der Gegenstände 1 bis 35 dadurch gekennzeichnet, dass das in Schritt a) eingesetzte Acetonäquivalent Aceton ist und das Molverhältnis von bei der Umsetzung eingesetztem Aceton zu Ammoniak in Schritt a) 3:1 bis 20:1 beträgt.
37. In einer weiteren Ausführungsform der Erfindung ist das Verfahren gemäß einem oder mehreren der Gegenstände 1 bis 36 dadurch gekennzeichnet, dass das in Schritt a) eingesetzte Acetonäquivalent Aceton ist und das Molverhältnis von bei der Umsetzung eingesetztem Aceton zu Ammoniak in Schritt a) 6:1 bis 9:1 beträgt.
38. In einer weiteren Ausführungsform der Erfindung ist das Verfahren gemäß einem oder mehreren der Gegenstände 1 bis 37 dadurch gekennzeichnet, dass die Umsetzung in Schritt a) bei Temperaturen im Bereich von 20 - 80°C durchgeführt wird.
39. In einer weiteren Ausführungsform der Erfindung ist das Verfahren gemäß einem oder mehreren der Gegenstände 1 bis 38 dadurch gekennzeichnet, dass die Umsetzung in Schritt a) bei Temperaturen im Bereich von 40 - 80°C durchgeführt wird.
40. In einer weiteren Ausführungsform der Erfindung ist das Verfahren gemäß einem oder mehreren der Gegenstände 1 bis 39 dadurch gekennzeichnet, dass die Umsetzung in Schritt a) bei Temperaturen im Bereich von 60 - 70°C durchgeführt wird.
41. In einer weiteren Ausführungsform der Erfindung ist das Verfahren gemäß einem oder mehreren der Gegenstände 1 bis 40 dadurch gekennzeichnet, dass die Umsetzung in Schritt a) bei einem Druck im Bereich von 1 bis 16 bar durchgeführt wird.
42. In einer weiteren Ausführungsform der Erfindung ist das Verfahren gemäß einem oder mehreren der Gegenstände 1 bis 41 dadurch gekennzeichnet, dass die Umsetzung in Schritt a) bei einem Druck im Bereich von 1 bis 10 bar durchgeführt wird.
43. In einer weiteren Ausführungsform der Erfindung ist das Verfahren gemäß einem oder mehreren der Gegenstände 1 bis 42 dadurch gekennzeichnet, dass die Umsetzung in Schritt a) bei einem Druck im Bereich von 1 bis 6 bar durchgeführt wird.
44. In einer weiteren Ausführungsform der Erfindung ist das Verfahren gemäß einem oder mehreren der Gegenstände 1 bis 43 dadurch gekennzeichnet, dass Ammoniak in Schritt a) gasförmig oder flüssig zugegeben wird.
45. In einer weiteren Ausführungsform der Erfindung ist das Verfahren gemäß einem oder mehreren der Gegenstände 2 bis 44 dadurch gekennzeichnet, dass in einem weiteren Schritt e) das extrahierte Triacetonamin-Rohprodukt aus Schritt d) destillativ zu Triacetonamin gereinigt wird.
46. In einer weiteren Ausführungsform der Erfindung ist das Verfahren gemäß einem oder mehreren der Gegenstände 1 bis 45 dadurch gekennzeichnet, dass der Katalysator in Schritt a) ausgewählt ist aus der Gruppe bestehend aus Brönstedt-Säuren, Ammoniumsalzen einer Brönstedtsäure, Phosphoniumsalzen einer Brönstedtsäure, Lewissäuren.
47. In einer weiteren Ausführungsform der Erfindung ist das Verfahren gemäß einem oder mehreren der Gegenstände 1 bis 46 dadurch gekennzeichnet, dass der Katalysator in Schritt a) eine Brönstedt-Säure ist.
48. In einer weiteren Ausführungsform der Erfindung ist das Verfahren gemäß einem oder mehreren der Gegenstände 1 bis 47 dadurch gekennzeichnet, dass der Katalysator in Schritt a) ausgewählt ist aus der Gruppe bestehend aus Salzsäure, Schwefelsäure, Salpetersäure, organische Säuren der Formel RCOOH, Sulfonsäuren der Formel RSO₃H, wobei R ausgewählt ist aus der Gruppe bestehend aus gesättigten, ungesättigten, verzweigten, unverzweigten, ringgeschlossenen, offenkettigen aliphatischen, aromatischen, substituierten, unsubstituierten Kohlenwasserstoffresten.
49. In einer weiteren Ausführungsform der Erfindung ist das Verfahren gemäß einem oder mehreren der Gegenstände 1 bis 46 dadurch gekennzeichnet, dass der Katalysator in Schritt a) ein Phosphoniumsalz einer Brönstedtsäure ist.
50. In einer weiteren Ausführungsform der Erfindung ist das Verfahren gemäß einem oder mehreren der Gegenstände 1 bis 46 und 49 dadurch gekennzeichnet, dass der Katalysator in Schritt a) ein Phosphoniumsalz einer Brönstedtsäure ist, wobei die Brönstedtsäure ausgewählt ist aus der Gruppe bestehend aus Salzsäure, Schwefelsäure, Salpetersäure, einer organischen Säuren der Formel RCOOH, Sulfonsäuren der Formel RSO₃H, wobei R ausgewählt ist aus der Gruppe bestehend aus gesättigten, ungesättigten, verzweigten, unverzweigten, ringgeschlossenen, offenkettigen aliphatischen, aromatischen, substituierten, unsubstituierten Kohlenwasserstoffresten.
51. In einer weiteren Ausführungsform der Erfindung ist das Verfahren gemäß einem oder mehreren der Gegenstände 1 bis 46 dadurch gekennzeichnet, dass der Katalysator in Schritt a) ein Ammoniumsalz einer Brönstedtsäure ist.
52. In einer weiteren Ausführungsform der Erfindung ist das Verfahren gemäß einem oder mehreren der Gegenstände 1 bis 46 und 51 dadurch gekennzeichnet, dass der Katalysator in Schritt a) ein Ammoniumsalz einer Brönstedtsäure ist, wobei die Brönstedtsäure ausgewählt ist aus der Gruppe bestehend aus Salzsäure, Schwefelsäure, Salpetersäure, einer organischen Säuren der Formel RCOOH, Sulfonsäuren der Formel RSO₃H, wobei R ausgewählt ist aus der Gruppe bestehend aus gesättigten, ungesättigten, verzweigten, unverzweigten, ringgeschlossenen, offenkettigen aliphatischen, aromatischen, substituierten, unsubstituierten Kohlenwasserstoffresten.
53. In einer weiteren Ausführungsform der Erfindung ist das Verfahren gemäß einem oder mehreren der Gegenstände 1 bis 46 und 51 bis 52 dadurch gekennzeichnet, dass der Katalysator in Schritt a) Ammoniumchlorid oder Ammoniumnitrat ist.
54. In einer weiteren Ausführungsform der Erfindung ist das Verfahren gemäß einem oder mehreren der Gegenstände 1 bis 46 und 51 bis 53 dadurch gekennzeichnet, dass der Katalysator in Schritt a) Ammoniumnitrat ist.
55. In einer weiteren Ausführungsform der Erfindung ist das Verfahren gemäß einem oder mehreren der Gegenstände 1 bis 46 dadurch gekennzeichnet, dass der Katalysator in Schritt a) eine Lewissäure ist.
56. In einer weiteren Ausführungsform der Erfindung ist das Verfahren gemäß einem oder mehreren der Gegenstände 1 bis 46 und 55 dadurch gekennzeichnet, dass der Katalysator in Schritt a) eine Lewissäure ist, wobei die Lewissäure ausgewählt ist aus Verbindungen der Elemente der vierten Gruppe des Periodensystems, Verbindungen der Elemente der dreizehnten Gruppe des Periodensystems, Salze von Alkalimetallen, Salze von Erdalkalimetallen.
57. In einer weiteren Ausführungsform der Erfindung ist das Verfahren gemäß einem oder mehreren der Gegenstände 1 bis 46 und 55 bis 56 dadurch gekennzeichnet, dass der Katalysator in Schritt a) eine Lewissäure ist, wobei die Lewissäure ausgewählt ist aus der Gruppe bestehend aus Halogeniden, Alkoxiden, Alkylverbindungen der Elemente der vierten und dreizehnten Gruppe des Periodensystems.
58. In einer weiteren Ausführungsform der Erfindung ist das Verfahren gemäß einem oder mehreren der Gegenstände 1 bis 46 und 55 bis 57 dadurch gekennzeichnet, dass der Katalysator in Schritt a) eine Lewissäure ist, wobei die Lewissäure ausgewählt ist aus der Gruppe bestehend aus AlCl₃, BF₃, TiCl₄, Al(OR)₃, B(OR)₃, Ti(OR)₄, AlR₃, wobei R ausgewählt ist aus der Gruppe bestehend aus gesättigten, ungesättigten, verzweigten, unverzweigten, ringgeschlossenen, offenkettigen aliphatischen, aromatischen, substituierten, unsubstituierten Kohlenwasserstoffresten.
59. In einer weiteren Ausführungsform der Erfindung ist das Verfahren gemäß einem oder mehreren der Gegenstände 1 bis 46 und 55 bis 56 dadurch gekennzeichnet, dass der Katalysator in Schritt a) eine Lewissäure ist, wobei die Lewissäure ausgewählt ist aus der Gruppe bestehend aus Salze von Alkalimetallen, Salze von Erdalkalimetallen.
60. In einer weiteren Ausführungsform der Erfindung ist das Verfahren gemäß einem oder mehreren der Gegenstände 1 bis 46 und 55 bis 56 und 59 dadurch gekennzeichnet, dass der Katalysator in Schritt a) eine Lewissäure ist, wobei die Lewissäure ausgewählt ist aus der Gruppe bestehend aus CaCl₂, MgCl₂, LiCl.
61. In einer weiteren Ausführungsform der Erfindung ist das Verfahren gemäß einem oder mehreren der Gegenstände 1 bis 60 dadurch gekennzeichnet, dass das Molverhältnis von Ammoniak zu Katalysator in Schritt a) im Bereich 1 : 0.8 bis 1 : 0.02 liegt.
62. In einer weiteren Ausführungsform der Erfindung ist das Verfahren gemäß einem oder mehreren der Gegenstände 1 bis 61 dadurch gekennzeichnet, dass das Molverhältnis von Ammoniak zu Katalysator in Schritt a) im Bereich 1 : 0.2 bis 1 : 0.05 liegt.
63. In einer weiteren Ausführungsform der Erfindung ist das Verfahren gemäß einem oder mehreren der Gegenstände 1 bis 62 dadurch gekennzeichnet, dass in Schritt d) als Hilfsstoff eine Base eingesetzt wird und die in Schritt d) eingesetzte Base und die in Schritt b) eingesetzte Base chemisch gleich sind.
64. In einer weiteren Ausführungsform der Erfindung ist das Verfahren gemäß einem oder mehreren der Gegenstände 1 bis 63 dadurch gekennzeichnet, dass das Lösungsmittel in Schritt a) ausgewählt ist aus der Gruppe bestehend aus aliphatischen Lösungsmitteln, bevorzugt Pentan, Hexan, Heptan, Octan, Decan, Cyclohexan, Tetramethylsilan; aromatischen Lösungsmitteln, bevorzugt Benzol, Toluol, Xylol; Ether-Verbindungen, bevorzugt Diethylether, Dipropylether, Dibutylether, Methyl-tert-Butylether; halogenierten Lösungsmitteln, bevorzugt Dichlormethan, Chloroform, Tetrachlormethan; Alkoholen, bevorzugt Methanol, Ethanol, Propanol, Isopropanol, Butanol, tert-Butanol; Estern, bevorzugt Methylacetat, Ethylacetat, Propylacetat, Butylacetat; Aceton.
65. In einer weiteren Ausführungsform der Erfindung ist das Verfahren gemäß einem oder mehreren der Gegenstände 1 bis 64 dadurch gekennzeichnet, dass das Lösungsmittel in Schritt a) Aceton ist.
66. In einer weiteren Ausführungsform der Erfindung ist das Verfahren gemäß einem oder mehreren der Gegenstände 1 bis 65 dadurch gekennzeichnet, dass die Extraktion in Schritt d) bei einer Temperatur von 20-80°C erfolgt.
67. In einer weiteren Ausführungsform der Erfindung ist das Verfahren gemäß einem oder mehreren der Gegenstände 1 bis 66 dadurch gekennzeichnet, dass die Extraktion in Schritt d) bei einer Temperatur mindestens 20°C, bevorzugt bei 20-99°C, besonders bevorzugt bei 45-90°C, ganz besonders bevorzugt bei 45-80°C, am bevorzugtesten bei einer Temperatur von 45 bis 65°C, und idealerweise bei einer Temperatur von 60°C erfolgt.
68. In einer weiteren Ausführungsform der Erfindung ist das Verfahren gemäß einem oder mehrerer der Gegenstände 1 bis 67 dadurch gekennzeichnet, dass das im Schritt c) erhaltene acetonabgereicherte Triacetonamin-Rohprodukt ein Acetongehalt von unter 20% (Massenprozent), bevorzugt von unter 15% (Massenprozent), besonders bevorzugt von unter 10% (Massenprozent), ganz besonders bevorzugt von unter 2.5% (Massenprozent), am bevorzugtesten von unter 1.5% (Massenprozent), idealerweise von unter 1% (Massenprozent) aufweist.
69. In einer weiteren Ausführungsform der Erfindung ist das Verfahren gemäß einem oder mehrerer der Gegenstände 1 bis 68 dadurch gekennzeichnet, dass die Extraktion des acetonabgereicherten Triacetonamin-Rohprodukts in Schritt d) als Kreuzstromextraktion durchgeführt wird.
70. In einer weiteren Ausführungsform der Erfindung ist das Verfahren gemäß einem oder mehrerer der Gegenstände 1 bis 69 dadurch gekennzeichnet, dass die Extraktion des acetonabgereicherten Triacetonamin-Rohprodukts in Schritt d) als Gegenstromextraktion durchgeführt wird.

### Detaillierte Beschreibung der Erfindung

Das erfindungsgemäße Verfahren eignet sich zur Herstellung von Triacetonamin, aber auch zur Herstellung von extrahiertem Triacetonamin-Rohprodukt. Im ersten Schritt [Schritt a)] des erfindungsgemäßen Verfahrens erfolgt die Umsetzung von Aceton mit einer ersten Base, ausgewählt aus Ammoniak in Gegenwart eines Katalysators, wobei ein Austragsgemisch erhalten wird, welches Aceton enthält. Die Reaktion kann kontinuierlich oder im Batch-Betrieb durchgeführt werden. Im Falle des Batch-Betriebs werden bevorzugt alle Edukte zusammengegeben, dann wird die Reaktionsmischung erwärmt. Als Reaktor kommen dabei alle üblichen Reaktortypen in Frage, z.B. Rührreaktoren, Schlaufenreaktoren oder Reaktoren mit innenliegenden Wärmetauschern.

Die Umsetzung kann in sämtlichen Lösungsmitteln stattfinden, die die Reaktion nicht verhindern. Insbesondere sind die möglichen Lösungsmittel aliphatische Lösungsmittel, bevorzugt Pentan, Hexan, Heptan, Octan, Decan, Cyclohexan, Tetramethylsilan; aromatische Lösungsmittel, bevorzugt Benzol, Toluol, Xylol; Ether-Verbindungen, bevorzugt Diethylether, Dipropylether, Dibutylether, Methyl-tert-Butylether; halogenierte Lösungsmittel, bevorzugt Dichlormethan, Chloroform, Tetrachlormethan; Alkohole, bevorzugt Methanol, Ethanol, Propanol, Isopropanol, Butanol, tert-Butanol; Ester, bevorzugt Methylacetat, Ethylacetat, Propylacetat, Butylacetat; Aceton.
Besonders bevorzugt findet die Reaktion in Aceton selbst statt.
Im kontinuierlichen Betrieb werden bevorzugt alle Chemikalien gleichzeitig bei der Reaktionstemperatur zudosiert. Bei der kontinuierlichen Reaktion kann jeder dem Fachmann bekannte Reaktor eingesetzt werden, z.B. ein kontinuierliches Strömungsrohr, ein kontinuierlicher Rührkessel, eine Rührkesselkaskade, sowie mögliche Kombinationen aus diesen einzelnen Elementen. Dabei wird bevorzugt eine Kombination von einem oder mehreren Reaktoren mit internem oder externem Kreislauf, gefolgt von einem Nachreaktor mit Strömungsrohrcharakteristik, eingesetzt.

Die Reaktionszeit im Batch-Betrieb liegt im Bereich von 1-15 Stunden, bevorzugt im Bereich von 3-9 Stunden, besonders bevorzugt im Bereich von 5-7 Stunden. Die Reaktionszeiten für die kontinuierliche Verfahrensführung ergeben sich durch die Gesamt-Verweilzeit der Reaktanden im Reaktor und liegen in dem für den Batch-Betrieb genannten Bereich.

Die Reaktion wird vorzugsweise bei erhöhter Temperatur durchgeführt, insbesondere bei Temperaturen im Bereich von 20 - 80°C, bevorzugt im Bereich von 40 - 80°C, besonders bevorzugt bei 60 - 70°C.

Die Reaktion kann entweder bei Eigendruck der Komponenten oder bei erhöhtem Druck durchgeführt werden. So kann die Reaktion im Bereich von 1-16 bar, bevorzugt im Bereich von 1-10 bar, besonders bevorzugt im Bereich von 1-6 bar durchgeführt werden.

Bei der Reaktion wird ein Acetonäquivalent direkt mit Ammoniak umgesetzt. Unter dem Begriff "Acetonäquivalent" im Sinne der Erfindung sind dabei Aceton selbst und Additions- oder Kondensationsprodukte von Aceton mit sich selbst (z.B. Mesityloxid, Diacetonalkohol, Phoron) und/oder Additions- oder Kondensationsprodukte aus Aceton mit Ammoniak (z.B. Diacetonamin, Acetonin) zu verstehen, welche ebenfalls produktiv zu TAA umgesetzt werden können. Insbesondere bedeutet der Begriff "Acetonäquivalent" im Sinne der Erfindung eine oder mehrere Verbindungen aus der Gruppe bestehend aus Mesityloxid, Diacetonalkohol, Phoron, Diacetonamin, Acetonin, Aceton. Bevorzugt bedeutet der Begriff "Acetonäquivalent" im Sinne der Erfindung Aceton. Zudem ist auch die Anwesenheit von inerten oder nicht-produktiven Bestandteilen in der Reaktionsmischung möglich.

Ammoniak wird bevorzugt als Reinstoff, d.h. als Gas zudosiert und liegt während der Reaktion gelöst in Aceton bzw. gelöst im Reaktionsgemisch vor.

Die Reaktion erfolgt in Gegenwart eines Katalysators. Dabei sind alle im Stand der Technik für diesen Reaktionstyp beschriebenen Katalysatoren geeignet, z.B. Brönstedt-Säuren, Salze dieser Säuren oder Lewissäuren. Der Begriff "Brönsted-Säuren" im Sinne der Erfindung umfasst insbesondere Salzsäure, Schwefelsäure, Salpetersäure, organische Säuren (RCOOH) oder Sulfonsäuren (RSO₃H) wobei R ausgewählt ist aus der Gruppe bestehend aus gesättigten, ungesättigten, verzweigten, unverzweigten, ringgeschlossenen, offenkettigen aliphatischen aromatischen, substituierten, unsubstituierten Kohlenwasserstoffresten. Substituierte Kohlenwasserstoffreste im Sinne der Erfindung sind Kohlenwasserstoffreste, die mit Heteroatomen substituiert sind, insbesondere Kohlenwasserstoffreste, welche mit einem oder mehreren -OH, -NH, -CN, Alkoxy- und/oder Halogenresten substituiert sind, bevorzugt mit einem oder mehreren Halogenresten substituiert sind, besonders bevorzugt mit einem oder mehreren Resten ausgewählt aus F, Cl, Br und/oder I substituiert sind, ganz besonders bevorzugt mit einem oder mehreren Resten ausgewählt aus F und/oder Cl substituiert sind. "Salze einer Brönstedtsäure" im Sinne der Erfindung sind insbesondere Ammoniumsalze (d.h. Salze mit Ammoniak, Aminen, Hydrazinen, Hydroxylaminen) oder Phosphoniumsalze (d.h. Salze mit Phosphanen). Lewis-Säuren im Sinne der Erfindung sind insbesondere Verbindungen der 4. bzw. der 13. Gruppe des Periodensystems, bevorzugt Halogenide (AlCl₃, BF₃, TiCl₄), Alkoxide [Al(OR)₃, B(OR)₃, Ti(OR)4] oder Alkylverbindungen (z.B. AlR₃ wobei R ausgewählt ist aus der Gruppe bestehend aus gesättigten, ungesättigten, verzweigten, unverzweigten, ringgeschlossenen, offenkettigen aliphatischen, aromatischen, substituierten, unsubstituierten Kohlenwasserstoffresten.
Lewis-Säuren im Sinne der Erfindung sind auch Salze Lewis-saurer Alkali- bzw. Erdalkalimetalle (z.B. CaCl₂, MgCl₂, LiCl).

Vorzugsweise ist der Katalysator ausgewählt aus der Gruppe der Ammoniumsalze, insbesondere aus der Gruppe umfassend Salze aus Ammoniak und starken Brönsted-Säuren [z.B. Salzsäure, Schwefelsäure, Salpetersäure, organische Säuren (RCOOH) oder Sulfonsäuren (RSO₃H), wobei R ausgewählt ist aus der Gruppe bestehend aus gesättigten, ungesättigten, verzweigten, unverzweigten, ringgeschlossenen, offenkettigen aliphatischen, aromatischen, substituierten, unsubstituierten Kohlenwasserstoffresten.

Ganz besonders bevorzugt wird Ammoniumnitrat als Katalysator bei der Umsetzung in Schritt a) eingesetzt. Ammoniumnitrat hat den Vorteil, dass es günstig, untoxisch, halogenidfrei und damit weniger korrosiv ist.

Die Einsatzverhältnisse der Edukte können in weiten Bereichen gewählt werden, insbesondere wird Aceton im Überschuss zu Ammoniak eingesetzt. Bevorzugt beträgt das Molverhältnis von eingesetztem Aceton zu Ammoniak. 3 : 1 bis 20 : 1, wobei ein Verhältnis von 6:1 bis 9:1 bevorzugt ist.

Der bei der Umsetzung eingesetzte Katalysator wird bevorzugt substöchiometrisch eingesetzt. Besonders bevorzugt liegt das Molverhältnis von Ammoniak zu Katalysator, vorzugsweise Ammoniumnitrat, im Bereich von 1 : 0.8 bis 1 : 0.02. Ganz besonders bevorzugt liegt das Molverhältnis von Aceton : Ammoniak : Ammoniumnitrat im Bereich von 7 - 8 : 0.9 - 1.1 : 0.085 - 0.098.

Als Ergebnis der im Schritt a) des Verfahrens erfolgten Umsetzung wird ein Austragsgemisch erhalten, welches in der Regel 20 bis 30% (Massenprozent) TAA, 50 bis 60% (Massenprozent) Aceton, 5 bis 15% (Massenprozent) Wasser, 10 bis 20% (Massenprozent) an organischen Nebenkomponenten und 0.5 bis 3% (Massenprozent) an Katalysator enthält, bezogen auf das Austragsgemisch, wobei die Summe der Anteil aller Komponenten 100% (Massenprozent) ergibt.

Der Begriff "Austragsgemisch" im Sinne der Erfindung bedeutet das Reaktionsgemisch, was nach erfolgter Umsetzung [Schritt a)] des erfindungsgemäßen Verfahrens erhalten wird und im folgenden Schritt b) mit einer Base umgesetzt wird.

Das in Schritt a) des Verfahrens erhaltene Austragsgemisch wird im nachfolgenden Schritt b) durch Zugabe einer weiteren Base deaktiviert, wobei ein Triacetonamin-Rohprodukt erhalten wird, welches Aceton enthält. Dabei beträgt die Menge der weiteren eingesetzten Base, bezogen auf die Menge des in Schritt a) des Verfahrens eingesetzten Katalysators, mindestens 1 molares Äquivalent. Die Menge der weiteren eingesetzten Base, bezogen auf die Menge des in Schritt a) des Verfahrens eingesetzten Katalysators, liegt bevorzugt im Bereich von 1.0 bis 2.0 molaren Äquivalenten, besonders bevorzugt im Bereich von 1.0 bis 1.4 molaren Äquivalenten, und beträgt am bevorzugtesten 1.2 molare Äquivalente.

Die Zugabe der weiteren Base in Schritt b) bewirkt eine Deaktivierung des Katalysators, wodurch unterbunden wird, dass sich das Triacetonamin, welches sich in dem nach Schritt a) erhaltene Austragsgemisch befindet, in den weiteren Verfahrensschritten zersetzt. Dabei führt die Zugabe der weiteren Base in Schritt b) erfindungsgemäß nicht zu einer hinreichenden Entfernung des deaktivierten Katalysators aus der organischen Phase, sondern dient lediglich der Deaktivierung des Katalysators.

Der Begriff "Deaktivierung" im Sinne der Erfindung bedeutet dabei, dass die katalytische Aktivität des in Schritt a) eingesetzten Katalysators gestoppt wird. Dies geschieht durch einer Base [M+B-, z.B. beschrieben in JP2003-206277; JP2001-031651; JP04154763; US4536581; G. Sosnovsky & M. Konieczny, Synthesis, 1976, 735-736; JP05140104; Plastic Additives 2006, 5(59), 46; A. Formenti & P. Piccinelli, Chimica e l'Industria, 2000, 82(5), 569-571]. Diese Base reagiert mit dem entsprechenden sauren Katalysator aus Schritt a), beispielsweise einem Ammoniumsalz (AH⁺X⁻) zu dem entsprechenden freien Amin (A), der konjugierten Saure (HB) sowie einem inerten Salz (M⁺X⁻). Wird z.B. die Deaktivierung von Ammoniumchlorid (AH⁺X⁻) mit NaOH (M⁺B⁻) betrachtet, so entstehen Ammoniak (A), Wasser (HB) und Natriumchlorid (M⁺X⁻).

"Weitere Base" im Sinne der Erfindung bedeutet insbesondere, dass es sich bei der im Schritt b) zugegebenen Base um eine von Ammoniak verschiedene Base handelt. Bevorzugt zeichnet sich die in Schritt b) eingesetzte weitere Base dadurch aus, dass sie eine stärkere Base als Ammoniak ist. "Stärkere Base als Ammoniak" heißt, dass die eingesetzte weitere Base einen geringeren pK_{B}-Wert als Ammoniak, bevorzugt einer mindestens 5% wässrige Lösung an Ammoniak besitzt.
Als weitere Base in Schritt b) des Verfahrens können generell alle dem Fachmann geläufigen Basen eingesetzt werden, welche diese Voraussetzungen erfüllen. Besonders bevorzugt kann die weitere Base ausgewählt werden aus der Gruppe bestehend aus Alkalimetallhydroxiden, Erdalkalimetallhydroxiden, Alkalimetallcarbonaten, Erdalkalimetallcarbonaten. Diese können einzeln oder in Kombination eingesetzt werden. Diese können in fester Form eingesetzt werden, da die organische Phase Wasser enthält, und feste Basen mit dem Reaktionswasser eine wässrige Phase ausbilden können. Die weitere Base kann aber auch in flüssiger Form eingesetzt werden. Beispielsweise kann sie als wässrige Lösung eingesetzt werden, wobei die Base bevorzugt in einer Konzentration von 40-60% (Massenprozent), besonders bevorzugt in einer Konzentration von 45-55% (Massenprozent) vorliegt. In einer weiteren Ausführungsform handelt es sich bei der Base um Natriumhydroxid (NaOH) oder Kaliumhydroxid (KOH), besonders bevorzugt um Natriumhydroxid.

KOH oder NaOH, bevorzugt NaOH, können dabei in fester Form eingesetzt werden. In einer weiteren vorteilhaften Ausführungsform der vorliegenden Erfindung werden KOH oder NaOH in flüssiger Form, beispielsweise gelöst in einem Lösungsmittel, insbesondere als wässrige Lösung, verwendet. Diese Ausführungsform ist besonders vorteilhaft für eine kontinuierliche Prozessführung. Dabei wird eine wässrige Lösung von KOH oder NaOH verwendet, wobei die Konzentration von KOH oder NaOH bevorzugt 40-60% (Massenprozent), besonders bevorzugt 45-55% (Massenprozent) beträgt. Ganz besonders bevorzugt wird dabei eine wässrige Lösung von NaOH verwendet, wobei die Konzentration von NaOH bevorzugt 40-60% (Massenprozent), besonders bevorzugt 45-55% (Massenprozent) beträgt.

Durch Umsetzung des in dem nach Schritt a) erhaltenen Austragsgemisches mit weiterer Base gemäß Schritt b) entsteht dann eine Lösung ("TAA-Rohprodukt"), welche eine organische und eine wässrige Phase aufweist. Die organische Phase enthält dabei das Triacetonamin. Die wässrige Phase kann im Rohprodukt belassen werden und der nachfolgende Schritt c) kann in Anwesenheit dieser wässrigen Phase durchgeführt werden.

Der Begriff "Triacetonamin-Rohprodukt" im Sinne der Erfindung bezeichnet dabei die Lösung, die entsteht, wenn in Schritt b) das Austragsgemisch aus Schritt a) mit einer weiteren Base versetzt wird. Diese Lösung weist eine wässrige und eine organische Phase auf.

In einer weiteren vorteilhaften Ausführungsform der vorliegenden Erfindung kann diese wässrige Phase vor Durchführung des folgenden Schrittes c) abgetrennt und im weiteren Verfahren nur die organische Phase ("organische Phase des TAA-Rohprodukts") verwendet werden. Es wurde überraschenderweise festgestellt, dass bei Abtrennung der wässrigen Phase im Anschluss an Schritt b) und Weiterverarbeitung der durch die Phasentrennung erhaltenen organischen Phase des TAA-Rohprodukts in den folgenden Schritten c) bis d) gemäß dem Verfahren der vorliegenden Erfindung die Stabilität an TAA erhöht wird. Dies trägt zusätzlich zu einer erhöhten Verfahrenseffizienz bei. Dies ist insbesondere dann vorteilhaft, wenn im Schritt b) mehr Base zugegeben wurde, als zur Deaktivierung des Katalysators nötig ist, d.h. mehr als 1 molares Äquivalent Base, bezogen auf die Menge des in Schritt a) eingesetzten Katalysators.

In dieser Ausführungsform [in welcher nach Zugabe der weiteren Base in Schritt b) erst eine Phasentrennung stattfindet und nur die erhaltene organische Phase des TAA-Rohprodukts im sich anschließenden Schritt c) verwendet wird] ist unter dem Begriff "Triacetonamin-Rohprodukt" in Schritt c) im Sinne der Erfindung die organische Phase des in Schritt b) erhaltenen Triacetonamin-Rohprodukts zu verstehen.

In Schritt c) des Verfahrens erfolgt die Entfernung von Aceton aus dem Triacetonamin-Rohprodukt aus Schritt b), wodurch ein acetonabgereichertes Triacetonamin-Rohprodukt erhalten wird. Dadurch erfolgt die Extraktion in Schritt d) nicht auf der Stufe des acetonhaltigen Triacetonamin-Rohprodukts, sondern erst nach der Entfernung des Acetons aus dem Triacetonamin-Rohprodukt. Es wurde in diesem Zusammenhang überraschenderweise gefunden, dass die Extraktion in Schritt d) nach Entfernung des Acetons deutlich effektiver verläuft.

"Entfernung von Aceton aus dem in Schritt b) erhaltenen Triacetonamin-Rohprodukt" im Sinne der Erfindung bedeutet dabei eine Entfernung des Acetons aus dem Triacetonamin-Rohprodukt bzw. der organischen Phase des Triacetonamin-Rohprodukts, so dass im resultierenden acetonabgereicherten Triacetonamin-Rohprodukt ein Acetongehalt von unter 40% (Massenprozent), insbesondere von unter 20% (Massenprozent), bevorzugt von unter 15% (Massenprozent), besonders bevorzugt von unter 10% (Massenprozent), ganz besonders bevorzugt von unter 2.5% (Massenprozent), am bevorzugtesten von unter 1.5% (Massenprozent), idealerweise von unter 1% (Massenprozent) vorliegt. In einer weiteren Ausführungsform ist der Acetongehalt im acetonabgereicherten Triacetonamin-Rohprodukt 20%, 19%, 18%, 17%, 16%, 15%, 14%, 13%, 12%, 11%, 10%, 9%, 8%, 7%, 6%, 5%, 4%, 3%, 2%, 1% (die Prozentangaben geben Massenprozent an), wobei der Acetongehalt umso bevorzugter ist, je kleiner er ist.

Die Entfernung des Acetons aus dem Triacetonamin-Rohprodukt kann insbesondere mittels Destillation erfolgen. Die destillative Entfernung von Aceton aus dem Triacetonamin-Rohprodukt kann z.B. in einer Destillation bei Normaldruck oder unter vermindertem Druck durchgeführt werden. Dabei können alle dem Fachmann bekannten Apparate verwendet werden, so z.B. ein gerührter Reaktor, ein Fallfilmverdampfer oder ein Dünnschichtverdampfer, jeweils in Kombination mit einer geeigneten Destillationskolonne oder einem anderen zur Destillation geeigneten Apparat. Die Destillation kann entweder diskontiniuierlich oder kontinuierlich erfolgen, wobei die exakten Destillationsbedingungen und Verweilzeiten so gewählt werden, dass der gewünschte Acetongehalt im Sumpf erreicht werden kann. Der bei der Destillation verwendete Druck liegt insbesondere im Bereich 0.4 - 2 bar.

Der Begriff "acetonabgereichertes Triacetonamin-Rohprodukt" im Sinne der Erfindung bezeichnet dabei die Lösung, die nach Durchführung von Schritt c) entsteht, wenn die Entfernung von Aceton aus dem Triacetonamin-Rohprodukt in Schritt c) des Verfahrens vorgenommen wird.

Die Besonderheit des erfindungsgemäßen Verfahrens liegt in Schritt d) des Verfahrens. In Schritt d) erfolgt die Extraktion des acetonabgereicherten Triacetonamin-Rohprodukts aus Schritt c) durch Zusatz eines Hilfsstoffs, wobei ein extrahiertes Triacetonamin-Rohprodukt erhalten wird. Der Begriff "extrahiertes Triacetonamin-Rohprodukt" bezeichnet im Sinne der Erfindung die im Schritt d) erhaltene organische Phase, welche das Triacetonamin enthält.

Es handelt sich bei dem Hilfsstoff um eine Base. Die in Schritt d) des Verfahrens eingesetzte Base entspricht dabei ganz besonders bevorzugt der in Schritt b) eingesetzten Base. "Entspricht" bedeutet im Sinne der Erfindung, dass in beiden Schritten b) und d) die chemisch gleiche Base eingesetzt wird. Beispielsweise wird in beiden Schritten b) und d) Natriumhydroxid eingesetzt.

Durch die erfindungsgemäße Verfahrensgestaltung erfolgt die Extraktion auf der Stufe des acetonabgereicherten Triacetonamin-Rohprodukts, was in Schritt c) erhalten wird, und nicht auf der Stufe des Triacetonamin-Rohproduktes, was in Schritt b) erhalten wird und noch acetonhaltig ist.
Es hat sich überraschend gezeigt, dass die Extraktion des acetonabgereicherten Triacetonamin-Rohprodukts deutlich effektiver erfolgt als jene des noch Aceton zu einem großen Anteil enthaltenden Triacetonamin-Rohprodukts. Dadurch kann die Menge an benötigter Base im Vergleich zum Stand der Technik deutlich reduziert werden. Zusätzlich ermöglicht dies die Verringerung der organischen Belastung der entstehenden wässrigen Phase. Als weiterer Vorteil ermöglicht dies die Verwendung flüssiger Base, z. B. wässriger NaOH, im Schritt d) des Verfahrens mit den vorab genannten Vorteilen. Eine direkte Entfernung des Acetons aus dem nach Schritt a) des Verfahrens erhaltenen Austragsgemisch ist wie beschrieben nicht möglich, da beispielsweise die bevorzugt zur Entfernung des Acetons vorgenommene Destillation in Anwesenheit des aktiven Katalysators zu einer mindestens teilweisen Zersetzung des Triacetonamins führen würde. Es wurde nun überraschend gefunden, dass eine Acetonentfernung aus dem Triacetonamin-Rohprodukt dennoch möglich ist, wenn der im Triacetonamin-Rohprodukt erhaltene Katalysator durch Zugabe von Base deaktiviert wird. Nach der im Schritt b) des Verfahrens erfolgten Deaktivierung des Katalysators kann dann im Schritt c) des Verfahrens die bevorzugt destillative Entfernung des Acetons aus dem Triacetonamin-Rohprodukt erfolgen. Nach Schritt c) des Verfahrens resultiert dann wie oben beschrieben das acetonabgereicherte Triacetonamin-Rohprodukt, das noch immer salzhaltig und wasserhaltig ist, selbst im Falle der oben beschriebenen Ausführungsform der Erfindung, in welcher zwischen den Schritten b) und c) des Verfahrens eine Phasentrennung vorgenommen und nur die organische Phase des Triacetonamin-Rohprodukts in Schritt c) des Verfahrens verwendet wird. In Schritt d) des Verfahrens wird dann die nach Schritt c) des Verfahrens erhaltene Lösung durch Zugabe weiterer Base entsalzt.

"Entsalzung" im Sinne der Erfindung bedeutet dabei, dass das sich bei der Deaktivierung des Katalysators in Schritt b) ergebende Salz aus der in Schritt c) erhaltenen Lösung entfernt wird, insbesondere bis auf einen Wert von unter 250 ppm, bevorzugt von unter 150 ppm, besonders bevorzugt von unter 100 ppm, ganz besonders bevorzugt von unter 50 ppm, am bevorzugtesten von unter 25 ppm, idealerweise von unter 1 ppm.

Die Menge an in Schritt d) zugesetzter Base liegt im Bereich von 1.0 bis 6.0 molaren Äquivalenten, bevorzugt im Bereich von 1.0 bis 4.0 molaren Äquivalenten, besonders bevorzugt im Bereich von 1.0 bis 2.0 molaren Äquivalenten, ganz besonders bevorzugt im Bereich von 1.0 bis 1.4 molaren Äquivalenten, bezogen auf die Menge des in Schritt a) des Verfahrens eingesetzten Katalysators. Am bevorzugtesten wird die Base in Schritt d) des Verfahrens in einer Menge von 1.2 molaren Äquivalenen bezogen auf die Menge des in Schritt a) des Verfahrens eingesetzten Katalysators, eingesetzt.

Als Base kann jede dem Fachmann geläufige Basen eingesetzt werden. Insbesondere kann die Base ausgewählt werden aus der Gruppe bestehend aus Alkalimetallhydroxiden, Erdalkalimetallhydroxiden, Alkalimetallcarbonaten und Erdalkalimetallcarbonaten. Diese können einzeln oder in Kombination eingesetzt werden. Diese können in fester Form eingesetzt werden. Sie können auch in flüssiger Form eingesetzt werden. Beispielsweise können sie als wässrige Lösung eingesetzt werden, wobei die Base bevorzugt in einer Konzentration von 40-60% (Massenprozent), besonders bevorzugt in einer Konzentration von 45-55% (Massenprozent) vorliegt.
Bevorzugt handelt es sich bei der Base um Natriumhydroxid (NaOH) oder Kaliumhydroxid (KOH), besonders bevorzugt um Natriumhydroxid. In einer weiteren vorteilhaften Ausführungsform der vorliegenden Erfindung werden KOH oder NaOH in flüssiger Form, beispielsweise gelöst in einem Lösungsmittel, bevorzugt als wässrige Lösung, verwendet. Diese Ausführungsform ist besonders vorteilhaft für eine kontinuierliche Prozessführung. Besonders bevorzugt wird dabei eine wässrige Lösung von KOH oder NaOH verwendet, wobei die Konzentration von KOH oder NaOH 40-60% (Massenprozent), besonders bevorzugt 45-55% (Massenprozent) beträgt. Ganz besonders bevorzugt wird dabei eine wässrige Lösung von NaOH verwendet, wobei die Konzentration von NaOH 40-60% (Massenprozent), besonders bevorzugt 45-55% (Massenprozent) beträgt.

Die Extraktion gemäß Schritt d) erfolgt bei einer Temperatur, bei welcher beide Phasen flüssig sind, insbesondere bei einer Temperatur von mindestens 20°C, bevorzugt bei 20-99°C, besonders bevorzugt bei 45-90°C, ganz besonders bevorzugt bei 45-80°C, am bevorzugtesten bei einer Temperatur von 45 bis 65°C, und idealerweise bei einer Temperatur von 60°C erfolgt.

Die Extraktion kann prinzipiell auf verschiedene Weisen erfolgen, z.B. kann die Extraktion sowohl in einer diskontinuierlichen als auch in einer kontinuierlichen Art und Weise erfolgen. Bei einer diskontinuierlichen Extraktion kann z.B. eine Mischung der aus Schritt c) erhaltenen Lösung mit der Base in einem Rührkessel, gefolgt von einer Phasentrennung in demselben oder einem zweiten Behälter erfolgen. In einer kontinuierlichen Ausführungsform kann die Extraktion durch Mischung der aus c) erhaltenen Lösung mit der Base, gepaart mit Phasentrennung in einem oder mehreren Mixer-Settler-Behältern oder einer oder mehrerer Extraktionskolonnen erfolgen.
Die erfindungsgemäßen Extraktionsmethoden sind beschrieben in W.R.A. Vauck, H.A. Müller, Grundoperationen chemischer Verfahrenstechnik, 11. Auflage, Seiten 787-805, wobei die Gegenstromextraktion insbesondere auf Seite 792-794 beschrieben ist, und die Kreuzstromextraktion insbesondere auf Seite 790-792 beschrieben ist.

Die Kreuzstromextraktion kann dabei in mindestens einer Stufe, bevorzugt in mindestens zwei Stufen, besonders bevorzugt mindestens drei, ganz besonders bevorzugt mindestens vier Stufen durchgeführt werden. Bei der Kreuzstromextraktion bezeichnet der Begriff "Stufe" dabei den einzelnen Vorgang, in dem eine mit zu extrahierendem Extraktstoff beladene Trägerflüssigkeit mit frischem Extraktionsmittel bis zum Einstellen des
Verteilungsgleichgewichts vermischt und darauf durch Absetzen abgetrennt wird.

Die Gegenstromextraktion kann dabei in mindestens einer theoretischen Trennstufe, bevorzugt in mindestens zwei theoretischen Trennstufen, besonders bevorzugt mindestens drei theoretischen Trennstufen, ganz besonders bevorzugt mindestens vier theoretischen Trennstufen durchgeführt werden. Bei der Gegenstromextraktion bezeichnet der Begriff "theoretische Trennstufe" das Vermischen zweier Flüssigphasen mit Extraktstoff-Übertragung und Trennen nach erzieltem Verteilungsgleichgewicht.

Ein optionaler Schritt e) des Verfahrens ist dadurch gekennzeichnet, dass das extrahierte Triacetonamin-Rohprodukt aus Schritt c) zu Triacetonamin gereinigt wird. Die Reinigung erfolgt bevorzugt destillativ. Dabei kann die Destillation entweder Batch-weise oder kontinuierlich erfolgen. Besonders bevorzugt ist eine kontinuierliche Destillation in mehreren Schritten, insbesondere in drei Schritten umfassend eine Mesityloxid/Wasser-Abtrennung, eine daran anschließende Mittelsieder-Abtrennung und eine abschließende TAA-Reindestillation.

Auch ohne weitere Ausführungen wird davon ausgegangen, dass ein Fachmann die obige Beschreibung im weitesten Umfang nutzen kann. Die bevorzugten Ausführungsformen und Beispiele sind deswegen lediglich als beschreibende, keinesfalls als in irgendeiner Weise limitierende Offenbarung aufzufassen.
Nachfolgend wird die vorliegende Erfindung anhand von Beispielen näher erläutert. Alternative Ausführungsformen der vorliegenden Erfindung sind in analoger Weise erhältlich.

### Beispiele

### 1. Allgemeine Vorschriften

### A) Umsetzung von Aceton mit Ammoniak in Gegenwart eines Katalysators, wobei ein Austragsgemisch erhalten wird

### A1) Herstellung von Austraasaemisch nach einem Batch- Verfahren - kleiner Maßstab

In einen 2 L Stahlautoklaven werden bei Raumtemperatur Aceton (1000 g, 17.2 mol) sowie Ammoniumnitrat (17.6 g, 0.220 mol) gegeben. Es wird Ammoniak (40.0 g, 2.35 mol) als Gas eindosiert, auf 65°C erwärmt und für sechs Stunden bei 65°C gerührt. Nach dem Abkühlen auf Raumtemperatur wird der Autoklav entspannt und das Rohprodukt (1050 g) wird ausgetragen. Man erhält eine homogenes Austragsgemisch mit der folgenden Zusammensetzung (Tabelle 1):

| **Tabelle 1:** Zusammensetzung des Austragsgemischs, welches in Beispiel A1 erhalten wird. Angaben in % (Massenprozent) | | | | | | | | | | |
|---|---|---|---|---|---|---|---|---|---|---|
| **Wasser** | **Ammonium -nitrat** | **Aceton** | **Mesityl-oxid** | **Diaceton-alkohol** | **Diaceton-amin** | **TMDH-Pyridin** | **Acetonin** | **Phoron** | **TAA** | **Rest** |
| 8.61 | 1.67 | 51.18 | 2.85 | 0.60 | 1.88 | 1.97 | 1.79 | 0.06 | 25.03 | 4.41 |

Das in Tabelle 1 beschriebene Austragsgemisch ist jenes, welches in den Beispielen 1-10 weiter verwendet wurde.

### A2) Herstellung von Austragsgemisch nach einem Batch-Verfahren - großer Maßstab

Es wird zusätzlich Austragsgemisch in einem größeren Maßstab hergestellt. Dazu werden in einem 90 L Stahlautoklaven bei Raumtemperatur Aceton (56.0 kg, 964 mol) sowie Ammoniumnitrat (990 g, 12.4 mol) gegeben. Es wird Ammoniak (2.24 kg, 132 mol) als Gas eindosiert, auf 65°C erwärmt und für sechs Stunden bei 65°C gerührt. Nach dem Abkühlen auf Raumtemperatur wird der Autoklav entspannt und das Rohprodukt (59 kg) wird ausgetragen. Man erhält eine homogenes Rohprodukt mit der folgenden Zusammensetzung (Tabelle 2):

| **Tabelle 2:** Zusammensetzung des Austragsgemischs, welches in Beispiel A2 erhalten wird. Angaben in % (Massenprozent) | | | | | | | | | | |
|---|---|---|---|---|---|---|---|---|---|---|
| **Wasser** | **Ammonium -nitrat** | **Aceton** | **Mesityl-oxid** | **Diaceton-alkohol** | **Diaceton-amin** | **TMDH-Pyridin** | **Acetonin** | **Phoron** | **TAA** | **Rest** |
| 9.34 | 1.63 | 50.23 | 2.15 | 0.55 | 1.69 | 2.04 | 0.84 | 0.06 | 27.39 | 4.08 |

### A3) Herstellung von Austragsgemisch nach einem kontinuierlichen Verfahren

Für das kontinuierliche Verfahren wird ein beheizbarer kontinuierlicher Rührkessel verwendet, in welchen Aceton, Ammoniak sowie eine Ammoniumnitratlösung kontinuierlich zudosiert werden können. Der Austrag aus dem Rührkessel wird durch einen separat beheizbaren Nachreaktor (1.6 L) geleitet, welcher eine Strömungsrohrcharakteristik besitzt. Zu Beginn der Reaktion wird der Rührkessel mit der gewünschten Menge an Edukten gefüllt und die gewünschten Temperaturen am Rührkessel sowie am Nachreaktor werden eingestellt. Die Eduktdosierung wird begonnen, und der Reaktionsaustrag aus dem Nachreaktor wird gesammelt. Dabei entspricht das molare Verhältnis der Edukte bei der Anfangsfüllung dem Verhältnis der Edukte bei der kontinuierlichen Dosierung. Es wird ein Eduktverhältnis von Aceton : Ammoniak = 7.33 : 1 und Ammoniak : Ammoniumnitrat = 10.6 : 1 gewählt.
In Abhängigkeit von den exakten Reaktionsparametern [Temperaturen, Verweilzeiten (VWZ) in den Reaktoren] wurden die in Tabelle 3 gezeigten Austragsgemische [es wurden 5 Versuche i) bis v) durchgeführt] erhalten:

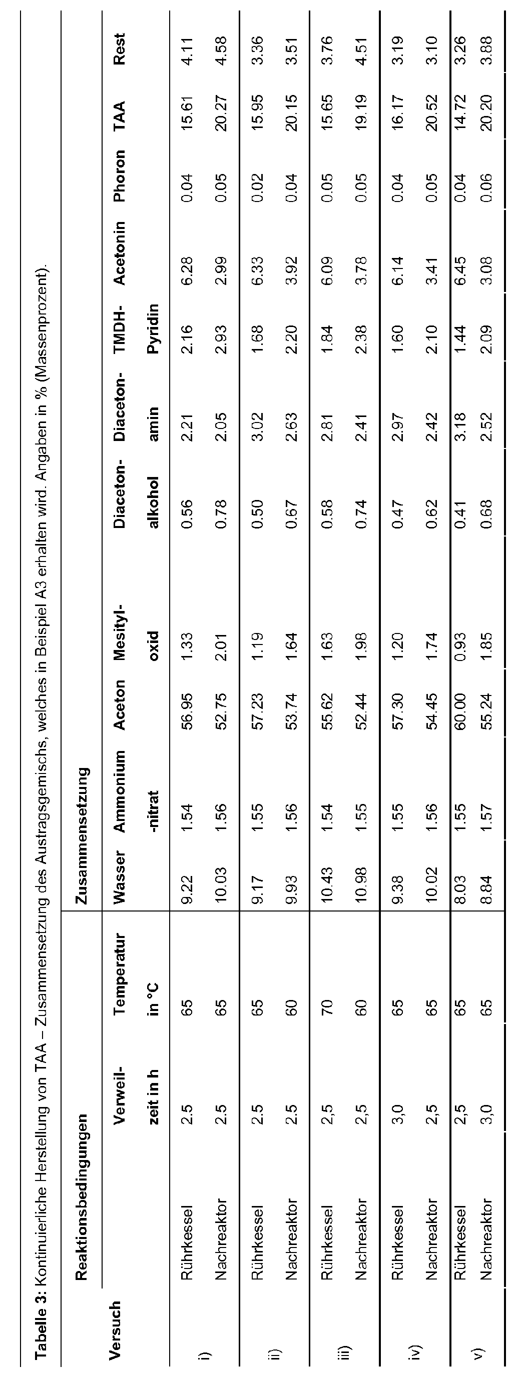

### B) Umsetzung des Austragsgemischs aus A) mit einer weiteren Base und Erhalt des Triacetonamin-Rohprodukts

Das Reaktionsprodukt aus A1) [1000 g, enthaltend 16.7 g (0,208 mol) Ammoniumnitrat, 512 g Aceton und 250 g TAA] wird mit 20.0 g einer 50% (Massenprozent) wässrigen Lösung von NaOH (0.250 mol; entsprechend 1.20 molaren Äquivalenten NaOH bezogen auf den Katalysator) versetzt. Die Mischung wird für 15 Minuten bei Raumtemperatur gerührt. Man erhält 1018 g eines zweiphasigen Triacetonamin-Rohprodukts, welches 250 g Triacetonamin enthält. Dieses besteht aus 1001 g einer organischen Phase (enthaltend 12.6 g Natriumnitrat, 510 g Aceton und 249 g TAA) sowie 17.0 g einer wässrigen Phase (enthaltend 4.9 g Natriumnitrat und 1.7 g Natriumhydroxid).

### C) Entfernung von Aceton aus dem Triacetonamin-Rohprodukt, wodurch ein acetonabgereichertes Triacetonamin-Rohprodukt erhalten wird

### C1) Entfernung von Aceton aus dem in B) erhaltenen Triacetonamin-Rohprodukt

Das zweiphasige Gemisch aus B) wird unter Normaldruck zum Sieden erhitzt. Das Aceton wird über eine Destillationskolonne (30cm-Glaskolonne mit Dampfteiler und Rückflusskühler) abgetrennt (Kopftemperatur 57-62°C).

### C2) Entfernung von Acetons in der bevorzugten Ausführungsform, dass die organische Phase des in B) erhaltenen Triacetonamin-Rohprodukts verwendet wird

Die organische Phase des in B) erhaltenen Triacetonamin-Rohprodukts wird abgetrennt. Diese wird dann unter Normaldruck zum Sieden erhitzt. Das Aceton wird über eine Destillationskolonne (30cm-Glaskolonne mit Dampfteiler und Rückflusskühler) abgetrennt (Kopftemperatur 57-62°C).

### D) Extraktion des acetonabgereicherten Triacetonamin-Rohprodukts aus Schritt C) durch Zusatz eines Hilfsstoffs und Erhalt des extrahierten Triacetonamin-Rohprodukts

### D1 Allgemeine Vorschrift für die Durchführung einer Kreuzstromextraktion (zwei- oder dreistufig)

Einsatzprodukt ist das acetonabgereicherte Rohprodukt aus C1 oder C2. Dieses wird bei 60°C in seine zwei Phasen getrennt (1. Extraktionsschritt). Die organische Phase wird mit Natriumhydroxid versetzt, die Mischung wird für 15 Minuten gerührt und die Phasen werden getrennt (2. Extraktionsschritt). Gegebenenfalls wird die organische Phase erneut mit Natriumhydroxid versetzt, die Mischung wird für 15 Minuten gerührt und die Phasen werden getrennt (3. Extraktionsschritt).

### D2) Allgemeine Vorschrift für die Durchführung einer Gegenstromextraktion dreistufig)

Einsatzprodukt ist das acetonabgereicherte Rohprodukt aus C1) oder C2). Zu diesem wird bei 60°C die wässrige Phase aus dem 2. Extraktionsschritt gegeben, die Mischung wird für 15 Minuten gerührt und die Phasen werden getrennt (1. Extraktionsschritt). Zu der resultierenden organischen Phase wird die wässrige Phase aus dem 3. Extraktionsschritt gegeben, die Mischung wird für 15 Minuten gerührt und die Phasen werden getrennt (2. Extraktionsschritt). Zu der resultierenden organischen Phase wird Natriumhydroxid gegeben, die Mischung wird für 15 Minuten gerührt und die Phasen werden getrennt (3. Extraktionsschritt). Die Zugabe der jeweiligen wässrigen Phase aus dem nachfolgenden Extraktionsschritt erfolgt dabei zwangsläufig aus einem vorher durchgeführten, analogen Experiment.

Analog zu dieser dreistufigen Gegenstromextraktion [wie in Beispielen G9), G10] kann auch eine zweistufige oder mehrstufige Gegenstromextraktion durchgeführt werden.

### E) Vergleichsbeispiele: Entfernung von Salzen aus dem Rohprodukt aus dem Austragsgemisch ohne vorherige Durchführung der Schritte B) und C)

### E1) Allgemeine Vorschrift für die Durchführung einer Kreuzstromextraktion ohne vorherige Durchführung der Schritte B) und C)

Einsatzprodukt ist der Austragsgemisch aus A1), A2) oder A3). Dies wird bei Raumtemperatur mit Natriumhydroxid versetzt, die Mischung wird für 15 Minuten gerührt und die Phasen werden getrennt (1. Extraktionsschritt). Die organische Phase wird ein zweites Mal mit Natriumhydroxid versetzt, die Mischung wird für 15 Minuten gerührt und die Phasen werden getrennt (2. Extraktionsschritt). Gegebenenfalls wird die organische Phase ein drittes Mal mit Natriumhydroxid versetzt, die Mischung wird für 15 Minuten gerührt und die Phasen werden getrennt (3. Extraktionsschritt).

### E2) Allgemeine Vorschrift für die Durchführung einer Gegenstromextraktion (dreistufig)

Einsatzprodukt ist der Reaktionsaustrag aus A1), A2) oder A3). Zu diesem wird bei Raumtemperatur die wässrige Phase aus dem 2. Extraktionsschritt gegeben, die Mischung wird für 15 Minuten gerührt und die Phasen werden getrennt (1. Extraktionsschritt). Zu der resultierenden organischen Phase wird die wässrige Phase aus dem 3. Extraktionsschritt gegeben, die Mischung wird für 15 Minuten gerührt und die Phasen werden getrennt (2. Extraktionsschritt). Zu der resultierenden organischen Phase wird Natriumhydroxid gegeben, die Mischung wird für 15 Minuten gerührt und die Phasen werden getrennt (3. Extraktionsschritt).

Die Zugabe der jeweiligen wässrigen Phase aus dem nachfolgenden Extraktionsschritt erfolgt dabei zwangsläufig aus einem vorher durchgeführten, analogen Experiment.
Analog zu dieser dreistufigen [wie in Beispielen G3), G4), G5)] Gegenstromextraktion kann auch eine zweistufige oder mehrstufige Gegenstromextraktion durchgeführt werden.

### F) Meßmethoden

### F1) Bestimmung des Nitratgehaltes

Die Bestimmung der Nitrationen erfolgte über lonenchromatographie gemäß DIN EN ISO 10304. Als Gerät wurde ein Metrohm lonenchromatograph mit Leitfähigkeitdetektion genutzt. Die verwendete Säule war eine Metrohm Metrosep Anion Dual 2. Als Eluent wurde eine wässrige Lösung von 2.0 mmol NaHCO₃, 1.3 mmol Na₂CO₃, 2% Aceton genutzt. Es wurden Injektionsschleifen von 10 µl bzw. 100 µl (für Proben mit einem Grenzwert < 20 mg/kg) genutzt. Der Kalibrierbereich der 10 µl-Schleife lag bei 0.5-100 mg/l. Der Kalibrierbereich der 100 µl-Schleife lag bei 0.05-2.0 mg/l.

### F2) Bestimmung des Gehaltes an organischen Komponenten

Die Bestimmung des Gehaltes aller organischen Komponenten wurde mit Gaschromatographie an einem Gaschromatograph HP 5890 durchgeführt. Die benutzte Säule war eine HP-50+, 30m x 0,25 mm x 0,25 mm. Als Trägergas wurde Stickstoff genutzt. Die Temperatur des Detektors lag bei 280°C, die Temperatur des Injektors lag bei 250°C. Das Temperaturprofil war 50°C für 2 min, dann Aufheizung mit einer Rate von 5°C / min auf 260°C (0 min).

### F3) Bestimmung des Gehaltes an Wasser

Der Wassergehalt wurde durch Karl-Fischer-Titration bestimmt an einem Mettler Toledo V20 Volumetric Karl-Fischer Titrator. Als Lösung wurde Hydranal Composite 5K /Medium K genutzt.

### G) Erfindungsgemäße Beispiele und Vergleichsbeispiele

### G1) Beispiel 1 (Vergleichsbeispiel; Dreifache Kreuzstromextraktion aus dem Austragsgemisch ohne Acetonentfernung)

1 kg des Austragsgemischs aus A1) [Tabelle 1; 250 g TAA, 1.67% (Massenprozent) NH₄NO₃] wird bei Raumtemperatur mit 20 g einer 50% (Massenprozent) wässrigen NaOH versetzt, 15 min gerührt und die Phasen werden getrennt (1.Extraktionsschritt). Es resultieren 1001 g einer organischen Phase OP1 [enthaltend 249 g TAA und 1.26% (Massenprozent) NaNO₃] und 17 g einer wässrigen Phase WP1 [enthaltend 28.8% (Massenprozent) NaNO₃]. OP1 wird erneut bei Raumtemperatur mit 20 g einer 50% (Massenprozent) wässrigen NaOH versetzt, 15 min gerührt und die Phasen werden getrennt (2. Extraktionsschritt). Es resultieren 960 g einer organischen Phase OP2 [enthaltend 248 g TAA und 0.53% (Massenprozent) NaNO₃] und eine wässrige Phase WP2 [enthaltend 16.6% (Massenprozent) NaNO₃]. OP2 wird dann bei Raumtemperatur ein drittes Mal mit 20 g einer 50% (Massenprozent) wässrigen NaOH versetzt, 15 min gerührt, und die Phasen werden getrennt (3. Extraktionsschritt). Es resultieren 932 g einer organischen Phase OP3 [enthaltend 244 g TAA und 1800 ppm NaNO₃] und eine wässrige Phase WP3 [enthaltend 7.9% (Massenprozent) NaNO₃].

Der TAA-Gehalt in OP3 wurde gaschromatographisch und ionenchromatographisch bestimmt zu 244 g TAA [97.6% bezogen auf den TAA-Gehalt im Austragsgemisch aus A1, gaschromatographisch bestimmt], Der Gehalt an NaNO₃ in OP3 betrug 1800 ppm (bestimmt über lonenchromatographie). Dies entspricht einem NaOH-Einsatz von 123 g NaOH pro kg aufgereinigtes TAA bzw. 3.61 molaren Äquivalenten bezogen auf den eingesetzten Ammoniumnitrat-Katalysator.

Beispiel 1 zeigt, dass bei nicht durchgeführter Acetonentfernung der Einsatz von 3.61 molaren Äquivalenten bezogen auf die Menge des in Schritt A1) eingesetzten

Ammoniumnitratkatalysators im Kreuzstromverfahren nicht ausreichen, um NaNO₃ vollständig aus der OP3 zu entfernen.

### G2) Beispiel 2 (Vergleichsbeispiel; Dreifache Kreuzstromextraktion aus dem Austragsgemisch ohne Acetonentfernung)

1 kg des Austragsgemischs aus A1) [Tabelle 1; 250 g TAA, 1.67% (Massenprozent) NH₄NO₃] wird bei Raumtemperatur mit 15 g fester NaOH versetzt, 15 min gerührt und die Phasen werden getrennt (1. Extraktionsschritt). Es resultieren 972 g einer organischen Phase OP1 [enthaltend 250 g TAA und 0.71% (Massenprozent) NaNO₃] und eine wässrige Phase WP1 [enthaltend 25.1% (Massenprozent) NaNO₃]. OP1 wird erneut bei Raumtemperatur mit 15 g fester NaOH versetzt, 15 min gerührt und die Phasen werden getrennt (2. Extraktionsschritt). Es resultieren 925 g einer organischen Phase OP2 [enthaltend 248 g TAA und 0.12% (Massenprozent) NaNO₃] und 56.1 g einer wässrigen Phase WP2 [enthaltend 10.3% (Massenprozent) NaNO₃]. OP2 wird dann bei Raumtemperatur ein drittes Mal mit 15 g fester NaOH versetzt, 15 min gerührt, und die Phasen werden getrennt (3. Extraktionsschritt). Es resultieren 895 g einer organischen Phase OP3 [enthaltend 247 g TAA, der Gehalt an NaNO₃ liegt unterhalb der Nachweisgrenze] und eine wässrige Phase WP3 [enthaltend 2.7% (Massenprozent) NaNO₃]. Der TAA-Gehalt in OP3 wurde bestimmt zu 247 g TAA [98.8% bezogen auf den TAA-Gehalt im Austragsgemisch aus A1, gaschromatographisch bestimmt]. Der Gehalt an NaNO₃ in OP3 lag unter der Nachweisgrenze von < 25 ppm; bestimmt über lonenchromatographie. Dies entspricht einem NaOH-Einsatz von 182 g NaOH pro kg aufgereinigtem TAA bzw. 5.41 molaren Äquivalenten bezogen auf den eingesetzten Ammoniumnitrat-Katalysator

Der Vergleich zwischen Beispiel 1 und 2 zeigt, dass bei Kreuzstromextraktion eine Abreicherung von NaNO₃ in OP3 bis unter die Nachweisgrenze ohne eine Verminderung des Acetongehalts im Triacetonamin-Rohprodukt nur unter Einsatz von 182 g NaOH pro kg aufgereinigtes TAA bzw. 5.4 molaren Äquivalenten [bezogen auf den in A1) eingesetzten Ammoniumnitrat-Katalysator] möglich ist. Zusätzlich muss NaOH in fester Form vorliegen.

### G3) Beispiel 3 (Vergleichsbeispiel; Dreifache Gegenstromextraktion aus dem Austragsgemisch ohne Acetonentfernung)

### 1. Durchgang:

Schritt 1/1): 1 kg des Austragsgemischs aus A1) {Tabelle 1; 250 g TAA, 16.7 g, [0.208 mol, 1.67% (Massenprozent)] NH₄NO₃} wird bei Raumtemperatur mit einer ersten simulierten wässrigen Phase SWP1 versetzt. SWP1 bestand aus 73.2 g Wasser, 14.8 g NaNO₃, 20.0 g NaOH. Nach Zugabe von SWP1 wurde 15 min bei Raumtemperatur gerührt und die erhaltene organische Phase OP1/1 wurde abgetrennt. Die erhaltene wässrige Phase WP1/1* wurde verworfen.
Schritt 1/2): OP1/1 wurde dann mit einer zweiten simulierten wässrigen Phase SWP2 versetzt. Diese zweite simulierte wässrige Phase SWP2 bestand aus 50.0 g Wasser, 6.1 g NaNO₃ und 20.0 g NaOH. Nach Zugabe von SWP2 wurde 15 min bei Raumtemperatur gerührt und die erhaltene organische Phase OP1/2 wurde von der wässrigen Phase WP1/2* abgetrennt. Die Phase WP1/2* wurde aufbewahrt.
Schritt 1/3): OP1/2 wurde dann mit 20 g fester NaOH versetzt. Die Mischung wurde 15 min bei Raumtemperatur gerührt und die erhaltene organische Phase OP1/3 wurde von der erhaltenen wässrigen Phase WP1/3* abgetrennt. Die Phase WP1/3* wurde aufbewahrt.
Schritt 1/4): Der Gehalt an TAA und NaNO₃ in OP1/3 wurde bestimmt (nicht in Tabelle 4 gezeigt).

### 2. Durchgang:

Schritt 2/1): Wiederholung von Schritt 1/1, nur dass nicht mit SWP1, sondern mit WP1/2* aus Schritt 1/2 extrahiert wurde. Es wurde eine neue organische Phase erhalten (OP2/1). Die resultierende wässrige Phase WP2/1* wurde verworfen.
Schritt 2/2): OP2/1 wurde dann mit WP1/3* aus Schritt 1/3 versetzt, 15 min bei Raumtemperatur gerührt. Es wurde eine neue organische Phase erhalten (OP2/2). Die resultierende wässrige Phase WP2/2* wurde aufbewahrt.
Schritt 2/3): Wiederholung von Schritt 1/3, nur dass anstatt OP1/2 die Phase OP2/2 verwendet wurde. Es wurde eine neue organische Phase OP2/3 erhalten. Die resultierende wässrige Phase WP2/3* wurde aufbewahrt.
Schritt 2/4): Der Gehalt an TAA und NaNO₃ in OP2/3 wurde bestimmt (nicht in Tabelle 4 gezeigt).

### 3. Durchgang:

Schritt 3/1): Wiederholung von Schritt 1/1, nur dass nicht mit SWP1, sondern mit WP2/2* aus Schritt 2/2 extrahiert wurde. Es wurde eine neue organische Phase erhalten (OP3/1), Die resultierende wässrige Phase WP3/1* wurde aufbewahrt. Der Gehalt an NaNO₃ und Triacetonamin in OP3/1 und WP3/1 * wurde bestimmt und ist in Tabelle 4 gezeigt.
Schritt 3/2): OP3/1, wurde dann mit WP2/3* aus Schritt 2/3 versetzt, 15 min bei Raumtemperatur gerührt. Es wurde eine neue organische Phase erhalten (OP3/2), Die resultierende wässrige Phase WP3/2* wurde aufbewahrt. Der Gehalt an NaNO₃ und Triacetonamin in OP3/2 und WP3/2* wurde bestimmt und ist in Tabelle 4 gezeigt.
Schritt 3/3): Wiederholung von Schritt 1/3, nur dass anstatt OP1/2 die Phase OP3/2 verwendet wurde. Es wurde eine neue organische Phase OP3/3 erhalten. Die resultierende wässrige Phase WP3/3* wurde aufbewahrt.
Schritt 3/4): Der Gehalt an TAA und NaNO₃ in OP3/3 und WP3/3* wurde bestimmt.

Die Gehalte an NaNO₃ und TAA in den organischen Phasen OP3/1, OP3/2 und OP3/3 wurden bestimmt und sind in Tabelle 4 gezeigt. Ebenso wurden die Gehalte an NaNO₃ und TAA in den wässrigen Phasen WP3/1*, WP3/2* und WP3/3* bestimmt und sind in der Tabelle 4 gezeigt.

Insgesamt wurde im dritten Durchgang des Beispiels 3 20 g NaOH (fest; 0.500 mol) zur Extraktion des in 1 kg des Austragsgemisches befindlichen Salzes NaNO₃ (0.208 mol) eingesetzt. Somit wurden 2.40 Moläquivalente NaOH pro im Austragsgemisch vorhandenen NaNO₃, welches wiederum direkt aus der Menge des eingesetzten Ammoniumnitratkatalysators resultiert, verbraucht. Auf die Menge des im Austragsgemisch vorhandenen TAA gerechnet beträgt die Menge des eingesetzten Natriumhydroxids 80 g NaOH/ kg TAA. In der erhaltenen organischen Phase OP3/3 wurde immer noch ein Wert von 800 ppm NaNO₃ festgestellt. Dies zeigt, dass diese Menge an NaOH bei der Gegenstromextraktion nicht ausreicht, um NaNO₃ bis unter die Nachweisgrenze aus der organischen Phase zu entfernen, wenn keine Acetonentfernung aus dem Triacetonamin-Rohprodukt vorgenommen wird.

### G4) Beispiel 4 (Vergleichsbeisoiel: Dreifache Gegenstromextraktion aus dem Austragsgemisch ohne Acetonentfernung)

### 1. Durchgang:

Schritt 1/1): 1 kg des Austragsgemischs aus A1) {Tabelle 1; 250 g TAA, 16.7 g, [0.208 mol, 1.67% (Massenprozent) NH₄NO₃]} wird bei Raumtemperatur mit einer ersten simulierten wässrigen Phase SWP1 versetzt. SWP1 bestand aus 89.3 g Wasser, 15.2 g NaNO₃, 30.0 g NaOH. Nach Zugabe von SWP1 wurde 15 min bei Raumtemperatur gerührt und die erhaltene organische Phase OP1/1 wurde abgetrennt. Die erhaltene wässrige Phase WP1/1* wurde verworfen.
Schritt 1/2): OP1/1 wurde dann mit einer zweiten simulierten wässrigen Phase SWP2 versetzt. Diese zweite simulierte wässrige Phase SWP2 bestand aus 62.5 g Wasser, 6.3 g NaNO₃ und 30.0 g NaOH. Nach Zugabe von SWP2 wurde 15 min bei Raumtemperatur gerührt und die erhaltene organische Phase OP1/2 wurde von der wässrigen Phase WP1/2* abgetrennt. Die Phase WP1/2* wurde aufbewahrt.
Schritt 1/3): OP1/2 wurde dann mit 30 g fester NaOH versetzt. Die Mischung wurde 15 min bei Raumtemperatur gerührt und die erhaltene organische Phase OP1/3 wurde von der erhaltenen wässrigen Phase WP1/3* abgetrennt. Die Phase WP1/3* wurde aufbewahrt.
Schritt 1/4): Der Gehalt an TAA und NaNO₃ in OP1/3 wurde bestimmt (nicht in Tabelle 4 gezeigt).

### 2. Durchgang:

Schritt 2/1): Wiederholung von Schritt 1/1, nur dass nicht mit SWP1, sondern mit WP1/2* aus Schritt 1/2 extrahiert wurde. Es wurde eine neue organische Phase erhalten (OP2/1). Die resultierende wässrige Phase WP2/1 * wurde verworfen.
Schritt 2/2): OP2/1 wurde dann mit WP1/3* aus Schritt 1/3 versetzt, 15 min bei Raumtemperatur gerührt. Es wurde eine neue organische Phase erhalten (OP2/2), Die resultierende wässrige Phase WP2/2* wurde aufbewahrt.
Schritt 2/3): Wiederholung von Schritt 1/3, nur dass anstatt OP1/2 die Phase OP2/2 verwendet wurde. Es wurde eine neue organische Phase OP2/3 erhalten. Die resultierende wässrige Phase WP2/3* wurde aufbewahrt.
Schritt 2/4): Der Gehalt an TAA und NaNO₃ in OP2/3 wurde bestimmt (nicht in Tabelle 4 gezeigt).

### 3. Durchgang:

Schritt 3/1): Wiederholung von Schritt 1/1, nur dass nicht mit SWP1, sondern mit WP2/2* aus Schritt 2/2 extrahiert wurde. Es wurde eine neue organische Phase erhalten (OP3/1). Die resultierende wässrige Phase WP3/1 * wurde aufbewahrt. Der Gehalt an NaNO₃ und TAA in OP3/1 und WP3/1 * wurde bestimmt und ist in Tabelle 4 gezeigt.
Schritt 3/2): OP3/1 wurde dann mit WP2/3* aus Schritt 2/3 versetzt, 15 min bei Raumtemperatur gerührt. Es wurde eine neue organische Phase erhalten (OP3/2). Die resultierende wässrige Phase WP3/2* wurde aufbewahrt. Der Gehalt an NaNO₃ und TAA in OP3/2 und WP3/2* wurde bestimmt und ist in Tabelle 4 gezeigt.
Schritt 3/3): Wiederholung von Schritt 1/3, nur dass anstatt OP1/2 die Phase OP3/2 verwendet wurde. Es wurde eine neue organische Phase OP3/3 erhalten. Die resultierende wässrige Phase WP3/3* wurde aufbewahrt.
Schritt 3/4): Der Gehalt an TAA und NaNO₃ in OP3/3 und WP3/3* wurde bestimmt.

Die Gehalte an NaNO₃ und TAA in den organischen Phasen OP3/1, OP3/2 und OP3/3 wurden bestimmt und sind in Tabelle 4 gezeigt. Ebenso wurden die Gehalte an NaNO₃ und TAA in den wässrigen Phasen WP3/1*, WP3/2* und WP3/3* bestimmt und sind in der Tabelle 4 gezeigt.

Insgesamt wurde im dritten Durchgang des Beispiels 4 30 g NaOH (fest; 0.750 mol) zur Extraktion des in 1 kg des Austragsgemisches befindlichen Salzes NaNO₃ (0.208 mol) eingesetzt. Somit wurden 3.61 Moläquivalente NaOH pro im Austragsgemisch vorhandenen NaNO₃, welches wiederum direkt aus der Menge des eingesetzten Ammoniumnitratkatalysators resultiert, verbraucht. Auf die Menge des aufgereinigten TAA gerechnet beträgt die Menge des eingesetzten NaOH 121 g NaOH/ kg TAA. In der erhaltenen organischen Phase OP3/3 wurde ein Wert an NaNO₃ festgestellt, der unterhalb der Nachweisgrenze von 25 ppm liegt. Dies zeigt, dass erst eine Menge von 3.6 molaren Äquivalenten NaOH (fest; bezogen auf die Menge des im Austragsgemisch vorhandenen Ammoniumnitratkatalysators) bei der Gegenstromextraktion ausreicht, um NaNO₃ bis unter die Nachweisgrenze aus der organischen Phase zu entfernen, wenn keine Acetonentfernung aus dem Triacetonamin-Rohprodukt vorgenommen wird.

### G5) Beispiel 5 (Vergleichsbeispiel; Dreifache Gegenstromextraktion aus dem Austragsgemisch ohne Acetonentfernung)

### 1. Durchgang:

Schritt 1/1): 1 kg des Austragsgemischs aus A1) {Tabelle 1; 250 g TAA, 16.7 g, [0.208 mol, 1.67% (Massenprozent)] NH₄NO₃} wird bei Raumtemperatur mit einer ersten simulierten wässrigen Phase SWP1 versetzt. SWP1 bestand aus 166.0 g Wasser, 8.5 g NaNO₃, 30.0 g NaOH. Nach Zugabe von SWP1 wurde 15 min bei Raumtemperatur gerührt und die erhaltene organische Phase OP1/1 wurde abgetrennt. Die erhaltene wässrige Phase WP1/1 * wurde verworfen.
Schritt 1/2): OP1/1 wurde dann mit einer zweiten simulierten wässrigen Phase SWP2 versetzt. Diese zweite simulierte wässrige Phase SWP2 bestand aus 76.0 g Wasser, 1.5 g NaNO₃ und 30.0 g NaOH. Nach Zugabe von SWP2 wurde 15 min bei Raumtemperatur gerührt und die erhaltene organische Phase OP1/2 wurde von der wässrigen Phase WP1/2* abgetrennt. Die Phase WP1/2* wurde aufbewahrt.
Schritt 1/3): OP1/2 wurde dann mit 60 g einer 50% (Massenprozent) wässrigen Lösung von NaOH versetzt. Die Mischung wurde 15 min bei Raumtemperatur gerührt und die erhaltene organische Phase OP1/3 wurde von der erhaltenen wässrigen Phase WP1/3* abgetrennt. Die Phase WP1/3* wurde aufbewahrt.
Schritt 1/4): Der Gehalt an TAA und NaNO₃ in OP1/3 wurde bestimmt (nicht in Tabelle 4 gezeigt).

### 2. Durchgang:

Schritt 2/1): Wiederholung von Schritt 1/1, nur dass nicht mit SWP1, sondern mit WP1/2* aus Schritt 1/2 extrahiert wurde. Es wurde eine neue organische Phase erhalten (OP2/1). Die resultierende wässrige Phase WP2/1 * wurde verworfen.
Schritt 2/2): OP2/1 wurde dann mit WP1/3* aus Schritt 1/3 versetzt, 15 min bei Raumtemperatur gerührt. Es wurde eine neue organische Phase erhalten (OP2/2). Die resultierende wässrige Phase WP2/2* wurde aufbewahrt.
Schritt 2/3): Wiederholung von Schritt 1/3, nur dass anstatt OP1/2 die Phase OP2/2 verwendet wurde. Es wurde eine neue organische Phase OP2/3 erhalten. Die resultierende wässrige Phase WP2/3* wurde aufbewahrt.
Schritt 2/4): Der Gehalt an TAA und NaNO₃ in OP2/3 wurde bestimmt (nicht in Tabelle 4 gezeigt).

### 3. Durchgang:

Schritt 3/1): Wiederholung von Schritt 1/1, nur dass nicht mit SWP1, sondern mit WP2/2* aus Schritt 2/2 extrahiert wurde. Es wurde eine neue organische Phase erhalten (OP3/1). Die resultierende wässrige Phase WP3/1 * wurde aufbewahrt. Der Gehalt an NaNO₃ und TAA in OP3/1 und WP3/1 * wurde bestimmt und ist in Tabelle 4 gezeigt.
Schritt 3/2): OP3/1 wurde dann mit WP2/3* aus Schritt 2/3 versetzt, 15 min bei Raumtemperatur gerührt. Es wurde eine neue organische Phase erhalten (OP3/2), Die resultierende wässrige Phase WP3/2* wurde aufbewahrt. Der Gehalt an NaNO₃ und TAA in OP3/2 und WP3/2* wurde bestimmt und ist in Tabelle 4 gezeigt.
Schritt 3/3): Wiederholung von Schritt 1/3, nur dass anstatt OP1/2 die Phase OP3/2 verwendet wurde. Es wurde eine neue organische Phase OP3/3 erhalten. Die resultierende wässrige Phase WP3/3* wurde aufbewahrt.
Schritt 3/4): Der Gehalt an TAA und NaNO₃ in OP3/3 wurde bestimmt.

Die Gehalte an NaNO₃ und TAA in den organischen Phasen OP3/1, OP3/2 und OP3/3 wurden bestimmt und sind in Tabelle 4 gezeigt. Ebenso wurden die Gehalte an NaNO₃ und TAA in den wässrigen Phasen WP3/1*, WP3/2* und WP3/3* bestimmt und sind in der Tabelle 4 gezeigt.

Insgesamt wurde im dritten Durchgang des Beispiels 5 30 g NaOH (0.750 mol) zur Extraktion des in 1 kg des Austragsgemisches befindlichen Salzes NaNO₃ (0.208 mol) eingesetzt. Im Gegensatz zu Beispiel 4 wurde diese allerdings in Form einer 50% (Massenprozent) wässrigen Lösung zugesetzt. Das entspricht somit einem Verhältnis von 3.61 molaren Äquivalenten NaOH pro im Austragsgemisch vorhandenen Ammoniumnitratkatalysator. Auf die Menge des im Austragsgemisch vorhandenen TAA gerechnet beträgt die Menge des eingesetzten NaOH 121 g NaOH/ kg TAA. In der erhaltenen organischen Phase OP3/3 wurde ein Wert von 600 ppm an NaNO₃ festgestellt. Dies zeigt, dass es in Vergleichsbeispiel 4 nur mit Einsatz von 3.6

Äquivalenten fester NaOH gelang, den Gehalt von NaNO₃ in der organischen Phase bis unter die Nachweisgrenze zu drücken.

### G6) Beispiel 6 (Erfindunasaemäßes Beispiel; Dreifache Kreuzstromextraktion aus dem acetonabgereicherten Triacetonamin-Rohprodukt mit Acetonentfernung)

Das Austragsgemisch aus A1) (1 kg, enthaltend 16.7 g (0.208 mol) Ammoniumnitrat, 512 g Aceton und 250 g TAA) wird mit 20 g einer 50% (Massenprozent) wässrigen Lösung von NaOH (10 g NaOH, 0.250 mol, 1.2 molare Äquivalente NaOH, bezogen auf den Ammoniumnitratkatalysator) versetzt. Die Mischung wird für 15 min bei Raumtemperatur gerührt und es wird ein Triacetonamin-Rohprodukt erhalten. Dieses wird einer Acetondestillation unterworfen. Dabei wird es unter Normaldruck zum Sieden erhitzt und das Aceton wird über eine Destillationskolonne (30cm-Glaskolonne mit Dampfteiler und Rückflusskühler) abgetrennt (Kopftemperatur 57 - 62°C).

Man erhält 420 g eines Destillats, welches neben Aceton Spuren an Wasser [0.9% (Massenprozent)] und 0.05% (Massenprozent) Mesityxloxid enthält. Als Rückstand erhält man 578 g eines zweiphasigen Gemisches, was 90 g Aceton und 240 g TAA enthält. Der Acetongehalt des acetonabgereicherten Triacetonamin-Rohprodukts beträgt somit 16% (Massenprozent).

Das zweiphasige Gemisch wird bei 60°C in seine Phasen getrennt, und man erhält 545 g einer organischen Phase OP1 [enthaltend 240 g TAA und 1.5% (Massenprozent) NaNO₃] und 30.9 g einer wässrigen Phase WP1 [enthaltend 26.3% (Massenprozent) NaNO₃]. Die organische Phase wird abgetrennt (1. Extraktionsschritt) und mit 20 g einer 50% (Massenprozent) wässrigen NaOH versetzt, 15 min gerührt und die Phasen werden getrennt (2. Extraktionsschritt). Es resultieren 501 g einer organischen Phase OP2 [enthaltend 237 g TAA und 0.33% (Massenprozent) NaNO₃] sowie 57.9 g einer wässrige Phase WP2 [enthaltend 16.2% (Massenprozent) NaNO₃]. OP2 wird erneut bei 60°C mit 20 g einer 50%

(Massenprozent) wässrigen NaOH versetzt, 15 min gerührt und die Phasen werden getrennt (3. Extraktionsschritt). Es resultieren 475 g einer organischen Phase OP3 (enthaltend 237 g TAA und 500 ppm NaNO₃) und 39.7 g einer wässrigen Phase WP3 [enthaltend 3.2% (Massenprozent) NaNO₃].

Der TAA-Gehalt in OP3 wurde gaschromatographisch und ionenchromatographisch bestimmt zu 244 g TAA [98.8% bezogen auf den TAA-Gehalt im Austragsgemisch A1, gaschromatographisch bestimmt]. Der Gehalt an NaNO₃ in OP3 betrug 500 ppm (bestimmt über lonenchromatographie). Dies entspricht einem NaOH-Einsatz von 127 g NaOH pro kg aufgereinigtes TAA bzw. 3.6 molare Äquivalente NaOH bezogen auf den eingesetzten Ammoniumnitrat-Katalysator. Dieses Beispiel belegt den Effekt der Acetonentfernung auf den Gehalt von NaNO₃ in der Lösung. Bei 16% Acetongehalt im zweiphasigen Destillationsrückstand verbleiben nach der Extraktion immer noch 500 ppm NaNO₃ in der organischen Phase OP3.

### G7) Beispiel 7 (Erfindungsgemäßes Beispiel; Dreifache Kreuzstromextraktion aus dem acetonabgereicherten Triacetonamin-Rohprodukt mit Acetonentfernung)

Das Austragsgemisch der TAA-Herstellung aus A1) [1 kg, enthaltend 16.7 g (0.208 mol) Ammoniumnitrat, 512 g Aceton und 250 g TAA] wird mit 20 g einer 50% (Massenprozent) wässrigen Lösung von NaOH (10 g NaOH, 0.250 mol, 1.2 molare Äquivalente NaOH, bezogen auf den Ammoniumnitratkatalysator) versetzt und es resultiert ein Triacetonamin-Rohprodukt. Die Mischung wird für 15 min bei Raumtemperatur gerührt. Das erhaltene Gemisch wird einer Acetondestillation unterworfen. Dabei wird es unter Normaldruck zum Sieden erhitzt und das Aceton wird über eine Destillationskolonne (30cm-Glaskolonne mit Dampfteiler und Rückflusskühler) abgetrennt (Kopftemperatur 57 - 62°C).

Man erhält 490 g eines Destillats, welches neben Aceton Spuren an Wasser [(1.1 % (Massenprozent)] und 0.3% (Massenprozent) Mesityxloxid enthält. Als Rückstand, dem acetonabgereichertem Triacetonamin-Rohprodukt, erhält man 528 g eines zweiphasigen Gemisches, was 19 g Aceton und 235 g TAA enthält. Der Acetongehalt des Rückstands beträgt somit 3.6% (Massenprozent).

Das zweiphasige Gemisch wird bei 60°C in seine Phasen getrennt, und man erhält 444 g einer organischen Phase OP1 [enthaltend 233 g TAA und 0.30% (Massenprozent) NaNO₃] und 80.4 g einer wässrigen Phase WP1 [enthaltend 26.3% (Massenprozent) NaNO₃]. Die organische Phase wird abgetrennt (1. Extraktionsschritt) und mit 20 g einer 50% (Massenprozent) wässrigen NaOH versetzt, 15 min gerührt und die Phasen werden getrennt (2. Extraktionsschritt). Es resultieren 417 g einer organischen Phase OP2 (enthaltend 232 g TAA und 300 ppm NaNO₃) sowie 42.3 g einer wässrige Phase WP2 [enthaltend 3.3% (Massenprozent) NaNO₃]. OP2 wird erneut bei 60°C mit 20 g einer 50% (Massenprozent) wässrigen NaOH versetzt, 15 min gerührt und die Phasen werden getrennt (3. Extraktionsschritt). Es resultieren 399 g einer organischen Phase OP3 (enthaltend 228 g TAA und < 25 ppm NaNO₃) und 31.5 g einer wässrigen Phase WP3 [enthaltend 0.3% (Massenprozent) NaNO₃].

Der TAA-Gehalt in OP3 wurde gaschromatographisch und ionenchromatographisch bestimmt. OP3 enthielt demnach 228 g TAA [97.0% bezogen auf den TAA-Gehalt im Austragsgemisch aus A1, gaschromatographisch bestimmt]. Der Gehalt an NaNO₃ in OP3 lag unterhalb der Nachweisgrenze von 25 ppm (bestimmt über lonenchromatographie). Dies entspricht einem NaOH-Einsatz von 132 g NaOH pro kg aufgereinigtes TAA bzw. 3.61 molare Äquivalente NaOH bezogen auf den eingesetzten Ammoniumnitrat-Katalysator. Dieses Beispiel belegt den Effekt der Acetonentfernung auf den Gehalt von NaNO₃ in der Lösung. Bei 16% Acetongehalt im zweiphasigen Destillationsrückstand verbleiben nach der Extraktion immer noch 500 ppm NaNO₃ in der organischen Phase OP3, während bei einer Senkung des Acetongehaltes auf 3.6% (Massenprozent) im acetonabgereicherten Triacetonamin-Rohprodukt bei sonst gleichbleibenden Bedingungen wie in diesem Beispiel der Anteil an NaNO₃ in der Phase OP3 bis unter die Nachweisgrenze sinkt.

### G8) Beispiel 8 (Erfindungsgemäßes Beispiel; Zweifache Kreuzstromextraktion aus dem acetonabgereicherten TAA-Rohprodukt)

Das Austragsgemisch der TAA-Herstellung aus A1 [1kg, enthaltend 16.7 g (0.208 mol) Ammoniumnitrat, 512 g Aceton und 250 g TAA] wird mit 20 g einer 50% (Massenprozent) wässrigen Lösung von NaOH (10 g NaOH, 0.250 mol, 1.2 molare Äquivalente NaOH, bezogen auf den Ammoniumnitratkatalysator) versetzt. Die Mischung wird für 15 min bei Raumtemperatur gerührt. Das erhaltene Gemisch wird einer Acetondestillation unterworfen. Dabei wird es unter Normaldruck zum Sieden erhitzt und das Aceton wird über eine Destillationskolonne abgetrennt (Kopftemperatur 57 - 62°C).

Man erhält 490 g eines Destillats, welches neben Aceton Spuren an Wasser [1.1 % (Massenprozent)] und 0.3% (Massenprozent) Mesityxloxid enthält. Als Rückstand erhält man 528 g eines zweiphasigen Gemisches, dem acetonabgereichertem Triacetonamin-Rohprodukt was 19 g Aceton und 235 g TAA enthält. Der Acetongehalt des Rückstands beträgt somit 3.6% (Massenprozent).

Das zweiphasige Gemisch wird bei 60°C in seine Phasen getrennt, und man erhält 441 g einer organischen Phase OP1 [enthaltend 234 g TAA und 0.27% (Massenprozent) NaNO₃] und 81 g einer wässrigen Phase WP1 [enthaltend 21.7% (Massenprozent) NaNO₃]. Die organische Phase wird abgetrennt (1. Extraktionsschritt) und mit 40 g einer 50% (Massenprozent) wässrigen NaOH versetzt, 15 min gerührt und die Phasen werden getrennt (2. Extraktionsschritt). Es resultieren 405 g einer organischen Phase OP2 (enthaltend 231 g TAA und 110 ppm NaNO₃) sowie 69.4 g einer wässrige Phase WP2 [enthaltend 1.92% (Massenprozent) NaNO₃].

Der TAA-Gehalt in OP2 wurde gaschromatographisch und ionenchromatographisch bestimmt. OP2 enthielt demnach 231 g TAA [98.3% bezogen auf den TAA-Gehalt im Austragsgemisch A1, gaschromatographisch bestimmt], Der Gehalt an NaNO₃ in OP2 lag bei 110 ppm (bestimmt über lonenchromatographie). Dies entspricht einem NaOH-Einsatz von 130 g NaOH pro kg aufgereinigtes TAA bzw. 3.61 molare Äquivalente NaOH bezogen auf den eingesetzten Ammoniumnitrat-Katalysator. Dieses Beispiel belegt, dass es zur vollständigen Entfernung von NaNO₃ aus der organischen Phase vorteilhaft ist, eine dreistufige Kreuzstromextraktion durchzuführen.

### G9) Beispiel 9 (Erfindungsgemäßes Beispiel; Dreifache Gegenstromextraktion aus dem Austragsgemisch mit Acetonentfernung)

Das Austragsgemisch aus A1) (1 kg, enthaltend 16.7 g (0.208 mol) Ammoniumnitrat, 512 g Aceton und 250 g TAA) wird mit 20 g einer 50% (Massenprozent) wässrigen Lösung von NaOH (10 g NaOH, 0.250 mol, 1.2 molare Äquivalente NaOH, bezogen auf den Ammoniumnitratkatalysator) versetzt. Dieses Triacetonamin-Rohprodukt wird für 15 min bei Raumtemperatur gerührt. Das erhaltene Gemisch wird einer Acetondestillation unterworfen. Dabei wird es unter Normaldruck zum Sieden erhitzt und das Aceton wird über eine Destillationskolonne abgetrennt (Kopftemperatur 57 - 62°C). Sobald kein Aceton mehr übergeht, wird die Destillation beendet. Man erhält 490 g eines Destillats, welches neben Aceton Spuren an Wasser [1.1 % (Massenprozent)] und 0.3% (Massenprozent) Mesityxloxid enthält. Als Rückstand erhält man 528 g eines zweiphasigen Gemisches, dem acetonabgereicherten Triacetonamin-Rohprodukt, was 19 g Aceton und 235 g TAA enthält. Der Acetongehalt des Rückstands beträgt somit 3.6% (Massenprozent). Der Rückstand wird dann einer Gegenstromextraktion, die bei 60° C durchgeführt wird, unterworfen, und es wird wie folgt vorgegangen:

### 1. Durchgang:

Schritt 1/1): 528 g des zweiphasigen Gemisches [235 g TAA, 17.7 g, (0.208 mol, 3.35 Massenprozent) NaNO₃] wird bei 60°C mit einer ersten simulierten wässrigen Phase SWP1 versetzt. SWP1 bestand aus 70.0 g Wasser, 14.0 g NaNO₃, 10.0 g NaOH. Nach Zugabe von SWP1 wurde 15 min gerührt und die erhaltene organische Phase OP1/1 wurde abgetrennt. Die erhaltene wässrige Phase WP1/1* wurde verworfen.
Schritt 1/2): OP1/1 wurde dann mit einer zweiten simulierten wässrigen Phase SWP2 versetzt. Diese zweite simulierte wässrige Phase SWP2 bestand aus 40.0 g Wasser, 6.0 g NaNO₃ und 10.0 g NaOH. Nach Zugabe von SWP2 wurde 15 min bei 60°C gerührt und die erhaltene organische Phase OP1/2 wurde von der wässrigen Phase WP1/2* abgetrennt. Die Phase WP1/2* wurde aufbewahrt.
Schritt 1/3): OP1/2 wurde dann mit 20 g einer 50% (Massenprozent) wässrigen Lösung NaOH versetzt. Die Mischung wurde 15 min bei 60°C gerührt und die erhaltene organische Phase OP1/3 wurde von der erhaltenen wässrigen Phase WP1/3* abgetrennt. Die Phase WP1/3* wurde aufbewahrt.
Schritt 1/4): Der Gehalt an TAA und NaNO₃ in OP1/3 wurde bestimmt (nicht in Tabelle 4 gezeigt).

### 2. Durchgang:

Schritt 2/1): Wiederholung von Schritt 1/1, nur dass nicht mit SWP1, sondern mit WP1/2* aus Schritt 1/2 extrahiert wurde. Es wurde eine neue organische Phase erhalten (OP2/1). Die resultierende wässrige Phase WP2/1 * wurde verworfen.
Schritt 2/2): OP2/1 wurde mit WP1/3* aus Schritt 1/3 extrahiert. Es wurde eine neue organische Phase erhalten (OP2/2). Die resultierende wässrige Phase WP2/2* wurde aufbewahrt.
Schritt 2/3): Wiederholung von Schritt 1/3, nur dass anstatt OP1/2 die Phase OP2/2 verwendet wurde. Es wurde eine neue organische Phase OP2/3 erhalten. Die resultierende wässrige Phase WP2/3* wurde aufbewahrt.
Schritt 2/4): Der Gehalt an TAA und NaNO₃ in OP2/3 wurde bestimmt (nicht in Tabelle 4 gezeigt).

### 3. Durchgang:

Schritt 3/1): Wiederholung von Schritt 1/1, nur dass nicht mit SWP1, sondern mit WP2/2* aus Schritt 2/2 extrahiert wurde. Es wurde eine neue organische Phase erhalten (OP3/1). Die resultierende wässrige Phase WP3/1 * wurde aufbewahrt. Der Gehalt an NaNO₃ und TAA in OP3/1 und WP3/1 * wurde bestimmt und ist in Tabelle 4 gezeigt.
Schritt 3/2): OP3/1 wurde mit WP2/3* aus Schritt 2/3 extrahiert wurde. Es wurde eine neue organische Phase erhalten (OP3/2), Die resultierende wässrige Phase WP3/2* wurde aufbewahrt. Der Gehalt an NaNO₃ und TAA in OP3/2 und WP3/2* wurde bestimmt und ist in Tabelle 4 gezeigt.
Schritt 3/3): Wiederholung von Schritt 1/3, nur dass anstatt OP1/2 die Phase OP3/2 verwendet wurde. Es wurde eine neue organische Phase OP3/3 erhalten. Die resultierende wässrige Phase WP3/3* wurde aufbewahrt.
Schritt 3/4): Der Gehalt an TAA und NaNO₃ in OP3/3 wurde bestimmt.

Die Gehalte an NaNO₃ und TAA in den organischen Phasen OP3/1, OP3/2 und OP3/3 wurden bestimmt und sind in Tabelle 4 gezeigt. Ebenso wurden die Gehalte an NaNO₃ und TAA in den wässrigen Phasen WP3/1*, WP3/2* und WP3/3* bestimmt und sind in der Tabelle 4 gezeigt.

Insgesamt wurde im dritten Durchgang des Beispiels 9 20 g NaOH [in Form einer 50% (Massenprozent) wässrigen Lösung; 0.500 mol] verwendet, wobei zur Deaktivierung 10 g NaOH und zur Extraktion des in 1 kg des Austragsgemisches befindlichen Salzes NH₄NO₃ (0.208 mol) nochmals 10 g NaOH eingesetzt wurden. Das entspricht somit einem Vrhältnis von 2.40 molaren Äquivalenten NaOH pro im Austragsgemisch vorhandenen NH₄NO₃. Auf die Menge des aus dem zweiphasigen Rückstand gewonnenen TAA (235 g) gerechnet beträgt die Menge des eingesetzten NaOH somit 87 g NaOH/ kg TAA. In der erhaltenen organischen Phase OP3/3 konnte kein NaNO₃ ermittelt werden, der Wert lag unterhalb der Nachweisgrenze, d.h. < 25 ppm. Dies zeigt, dass bei einer Acetonentfernung aus dem TAA-Rohprdukt und Extraktion des acetonabgereicherten TAA-Rohprodukts mit einem Acetongehalt von 3.6% (Massenprozent) die Menge von 2.40 Äquivalenten NaOH, bezogen auf die Menge des im Austragsgemisch vorhandenen NaNO₃ ausreicht, um NaNO₃ bis unter die Nachweisgrenze aus der organischen Phase zu entfernen.

### G10) Beispiel 10 (Erfindungsgemäßes Beispiel; Dreifache Gegenstromextraktion aus dem Austragsgemisch mit Acetonentfernung und unter Verwendung der organischen Phase des Triacetonamin-Rohprodukts)

Das Austragsgemisch aus A1) [1 kg, enthaltend 16.7 g (0.208 mol) Ammoniumnitrat, 512 g Aceton und 250 g TAA] wird mit 20 g einer 50% (Massenprozent) wässrigen Lösung von NaOH (10 g NaOH, 0.250 mol, 1.2 molare Äquivalente NaOH, bezogen auf den Ammoniumnitratkatalysator) versetzt. Die Mischung wird für 15 min bei Raumtemperatur gerührt. Man erhält 1001 g einer organischen Phase (enthaltend 12.6 g NaNO₃, 510 g Aceton, 249 g TAA) sowie 17.0 g einer wässrigen Phase (enthaltend 4.9 g NaNO₃, 1.7 g NaOH). Die organische Phase des Triacetonamin-Rohprodukts wird von der wässrigen Phase abgetrennt und unter Normaldruck zum Sieden erhitzt. Das Aceton wird über eine Destillationskolonne abgetrennt (Kopftemperatur 57 - 62°C). Sobald kein Aceton mehr übergeht, wird die Destillation beendet. Man erhält 488 g eines Destillats, welches neben Aceton Spuren an Wasser [0.9% (Massenprozent)] auch 0.3% (Massenprozent) Mesityxloxid enthält. Als Rückstand erhält man 531 g eines zweiphasigen Gemisches, was 22 g Aceton und 247 g TAA enthält. Der Acetongehalt des Rückstands beträgt somit 4.1% (Massenprozent).

Der Rückstand wird dann einer Gegenstromextraktion unterworfen, die bei 60°C durchgeführt wird, und es wird wie folgt vorgegangen:

### 1. Durchgang:

Schritt 1/1): 531 g des zweiphasigen Gemisches [247 g TAA, 17.7 g (0.208 mol, 3.35 Massenprozent) NaNO₃] wird bei 60°C mit einer ersten simulierten wässrigen Phase SWP1 versetzt. SWP1 bestand aus 60.0 g Wasser, 4.6 g NaNO₃, 10.0 g NaOH. Nach Zugabe von SWP1 wurde 15 min gerührt und die erhaltene organische Phase OP1/1 wurde abgetrennt. Die erhaltene wässrige Phase WP1/1* wurde verworfen.
Schritt 1/2) OP1/1 wurde dann mit einer zweiten simulierten wässrigen Phase SWP2 versetzt. Diese zweite simulierte wässrige Phase SWP2 bestand aus 30.0 g Wasser, 0.6 g NaNO₃ und 10.0 g NaOH. Nach Zugabe von SWP2 wurde 15 min bei 60°C gerührt und die erhaltene organische Phase OP1/2 wurde von der wässrigen Phase WP1/2* abgetrennt. Die Phase WP1/2* wurde aufbewahrt.
Schritt 1/3) OP1/2 wurde dann mit 20 g einer 50% (Massenprozent) wässrigen Lösung NaOH versetzt. Die Mischung wurde 15 min bei 60°C gerührt und die erhaltene organische Phase OP1/3 wurde von der erhaltenen wässrigen Phase WP1/3* abgetrennt. Die Phase WP1/3* wurde aufbewahrt.
Schritt 1/4): Der Gehalt an TAA und NaNO₃ in OP1/3 wurde bestimmt (nicht in Tabelle 4 gezeigt).

### 2. Durchgang:

Schritt 2/1): Wiederholung von Schritt 1/1, nur dass nicht mit SWP1, sondern mit WP1/2* aus Schritt 1/2 extrahiert wurde. Es wurde eine neue organische Phase erhalten (OP2/1). Die resultierende wässrige Phase WP2/1 * wurde verworfen.
Schritt 2/2): OP2/1 wurde mit WP1/3* aus Schritt 1/3 extrahiert. Es wurde eine neue organische Phase erhalten (OP2/2). Die resultierende wässrige Phase WP2/2* wurde aufbewahrt.
Schritt 2/3): Wiederholung von Schritt 1/3, nur dass anstatt OP1/2 die Phase OP2/2 verwendet wurde. Es wurde eine neue organische Phase OP2/3 erhalten. Die resultierende wässrige Phase WP2/3* wurde aufbewahrt.
Schritt 2/4): Der Gehalt an TAA und NaNO₃ in OP2/3 wurde bestimmt (nicht in Tabelle 4 gezeigt).

### 3. Durchgang:

Schritt 3/1): Wiederholung von Schritt 1/1, nur dass nicht mit SWP1, sondern mit WP2/2* aus Schritt 2/2 extrahiert wurde. Es wurde eine neue organische Phase erhalten (OP3/1). Die resultierende wässrige Phase WP3/1 * wurde aufbewahrt. Der Gehalt an NaNO₃ und TAA in OP3/1 und WP3/1 * wurde bestimmt und ist in Tabelle 4 gezeigt.
Schritt 3/2): OP3/1 wurde mit WP2/3* aus Schritt 2/3 extrahiert. Es wurde eine neue organische Phase erhalten (OP3/2). Die resultierende wässrige Phase WP3/2* wurde aufbewahrt. Der Gehalt an NaNO₃ und TAA in OP3/2 und WP3/2* wurde bestimmt und ist in Tabelle 4 gezeigt. Schritt 3/3): Wiederholung von Schritt 1/3, nur dass anstatt OP1/2 die Phase OP3/2 verwendet wurde. Es wurde eine neue organische Phase OP3/3 erhalten. Die resultierende wässrige Phase WP3/3* wurde aufbewahrt.
Schritt 3/4): Der Gehalt an TAA und NaNO₃ in OP3/3 wurde bestimmt.

Die Gehalte an NaNO₃ und TAA in den organischen Phasen OP3/1, OP3/2 und OP3/3 wurden bestimmt und sind in Tabelle 4 gezeigt. Ebenso wurden die Gehalte an NaNO₃ und TAA in den wässrigen Phasen WP3/1*, WP3/2* und WP3/3* bestimmt und sind in der Tabelle 4 gezeigt.

Insgesamt wurde im dritten Durchgang des Beispiels 10 20 g NaOH [in Form einer 50% (Massenprozent) wässrigen Lösung; 0.500 mol] verwendet, wobei zur Deaktivierung 10 g NaOH und zur Extraktion des in 1 kg des Austragsgemisches befindlichen Salzes NaNO₃ (0.208 mol) nochmals 10 g eingesetzt wurden. Das entspricht somit einem Molverhältnis von 2.40 molaren Äquivalenten NaOH pro im Austragsgemisch vorhandenen NaNO₃, welches wiederum direkt aus der Menge des eingesetzten Ammoniumnitratkatalysators resultiert. Auf die Menge des aus dem zweiphasigen Rückstand gewonnenen TAA (235 g) gerechnet beträgt die Menge des eingesetzten NaOH somit 82 g NaOH/ kg TAA. In der erhaltenen organischen Phase OP3/3 konnte kein NaNO₃ ermittelt werden, der Wert lag unterhalb der Nachweisgrenze, d.h. < 25 ppm. Dies zeigt, dass bei einer Verwendung der organischen Phase des Triacetonaminrohprodukts und der Acetonentfernung aus der organischen Phase des Triacetonamin-Rohprodukts unter Verringerung des Acetongehalts auf 4.1% (Massenprozent) die Menge von 2.40 Äquivalenten NaOH, bezogen auf die Menge des im Austragsgemisch vorhandenen NaNO₃ ausreicht, um NaNO₃ bis unter die Nachweisgrenze aus der organischen Phase OP3 zu entfernen.
Zusätzlich zeigt der Vergleich von Vergleichsbeispielen 9 und 10, dass die TAA-Ausbeute, bezogen auf den Austrag aus der TAA-Synthese, (97.9% verglichen mit 99.2% in Vergleichsbeispiel 9 bzw. 10) höher liegt, wenn vor der Gegenstromextraktion eine Phasentrennung vorgenommen wird und nur die organische Phase der Gegenstromextraktion unterworfen wird.

Bei allen erfindungsgemäßen Beispielen, bei denen eine Gegenstromextraktion vorgenommen wurde (Beispiel 9 und 10) lag die Menge des eingesetzten NaOH bezogen auf das erhaltene TAA bei 87 bzw. 82 [g eingesetzter NaOH pro kg gewonnenem Triacetonamin]. Demgegenüber liegt dieser Wert im Gegenstromverfahren nach JP2003-206277 bei 169 [g TAA/ 1 kg TAA]. Dieses Verfahren ist in Tabelle 4 als Beispiel 11 aufgeführt. Diese Ergebnisse zeigen, dass das erfindungsgemäße Verfahren verglichen mit jenem in JP2003-06277 beschriebenem den überraschenden Vorteil bietet, dass eine Entfernung von Salzen aus der organischen Phase unter Einsatz nur der Hälfte der Menge an NaOH möglich ist.

Dieses überraschende Ergebnis zeigt sich auch im Vergleich mit den Beispielen 1 bis 5. So ist es bei einer Gegenstromextraktion nach Beispiel 4 nicht möglich, bei Einsatz von 2.4 molaren Äquivalenten NaOH (bezogen auf den im Austragsgemisch vorhandenen Ammoniumnitrat-Katalysator) NaNO₃ bis unter die Nachweisgrenze aus der organischen Phase OP3 zu eliminieren. Es verbleiben 800 ppm Restgehalt. Demgegenüber ist es in Beispielen 9 und 10 möglich, mit Einsatz von 2.4 molaren Äquivalenten NaOH (bezogen auf den im Austragsgemisch vorhandenen Ammoniumnitrat-Katalysator) den NaNO₃-Gehalt der organischen Phase bis unter die Nachweisgrenze zu reduzieren.
Ohne Acetonentfernung gelingt dies nur unter Einsatz von 3.61 molaren Äquivalenten NaOH (bezogen auf den im Austragsgemisch vorhandenen Ammoniumnitrat-Katalysator) und dies auch nur dann, wenn feste NaOH eingesetzt wird (Beispiel 4). 2.4 molare Äquivalente NaOH (bezogen auf den im Austragsgemisch vorhandenen Ammoniumnitrat-Katalysator) genügen nicht (Beispiel 3). Bei Verwendung einer wässrigen Lösung von NaOH (Beispiel 5) gelingt die Reduzierung von NaNO₃ in der organischen Phase OP3 nicht einmal mit 3.61 molaren Äquivalenten NaOH bezogen auf den im Austragsgemisch vorhandenen Ammoniumnitrat-Katalysator. Wie im Vergleich mit den Beispielen 1 bis 5 deutlich wird, ist es durch das erfindungsgemäße Verfahren außerdem möglich, den Einsatz von NaOH [bezogen auf den in Schritt A1) verwendeten Ammoniumnitrat-Katalysator] deutlich zu verringern.

Aus dem Vergleich von Beispiel 7 mit den Beispielen 1 und 2 geht hervor, dass es bei der Kreuzstromextraktion ohne Acetonentfernung nur unter Einsatz von 5.41 molaren Äquivalenten NaOH, eingesetzt als feste NaOH, bezogen auf den im Austragsgemisch vorhandenen Ammoniumnitrat-Katalysator gelingt, den NaNO₃ in der organischen Phase OP3 unter die Nachweisgrenze zu reduzieren (Beispiel 2). Bei Einsatz von 3.61 molaren Äquivalenten NaOH, eingesetzt als wässrige Lösung, bezogen auf den im Austragsgemisch vorhandenen Ammoniumnitrat-Katalysator gelingt dies nicht (Beispiel 1).

Somit ermöglicht das erfindungsgemäße Verfahren eine effizientere Entfernung von Salzen aus der organischen Phase, da diese unter Einsatz einer deutlich reduzierten Menge an NaOH abläuft. Dies erlaubt eine beträchtliche Einsparung an Material und löst damit die Aufgabe der vorliegenden Erfindung. Die Ergebnisse sind in Tabelle 4 zusammengefasst.

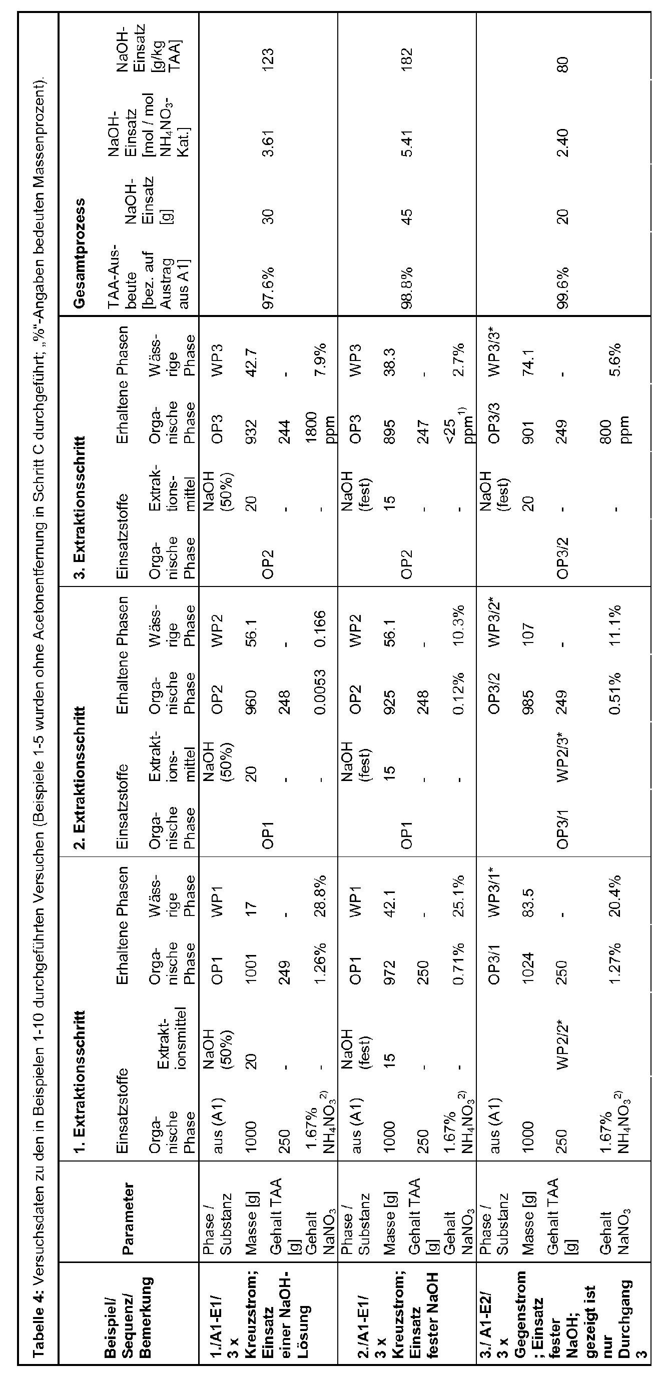

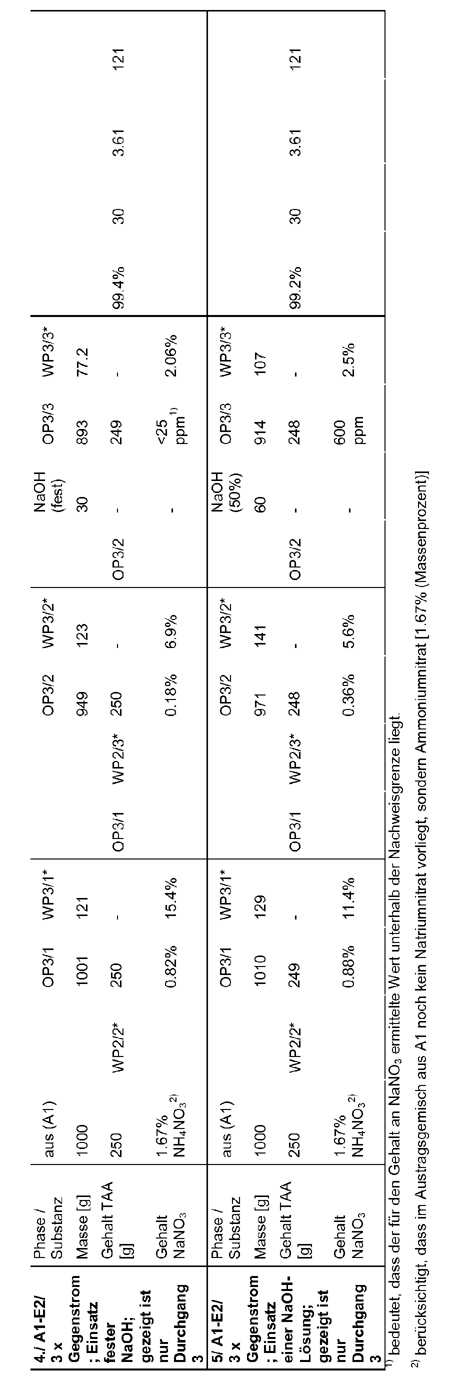

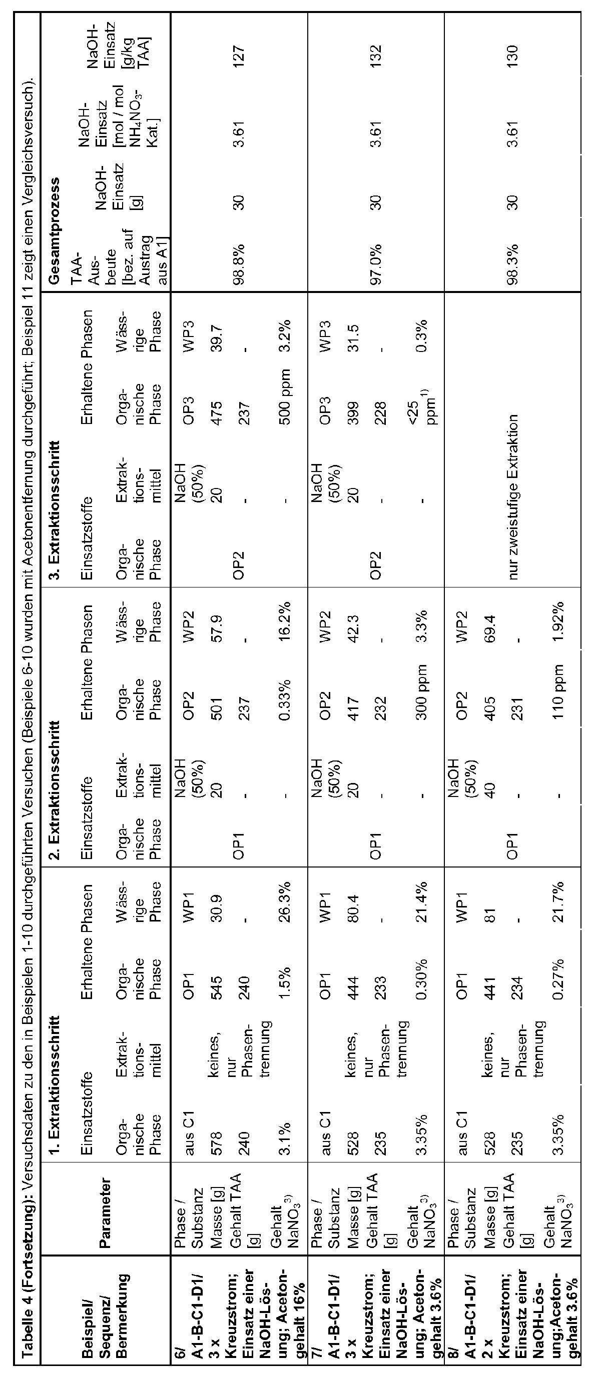

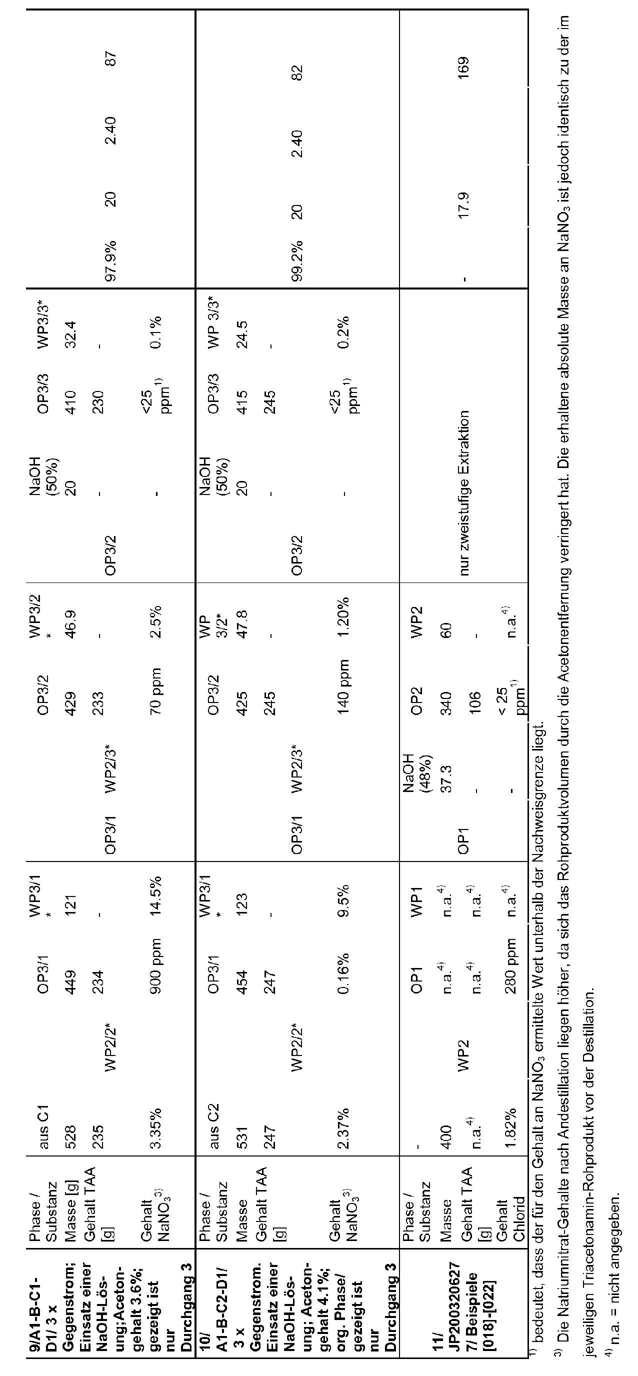

### H) Reinigung des extrahierten Triacetonamin-Rohprodukts

Ein extrahiertes Triacetonamin-Rohprodukt entsprechend der organischen Phase OP3/3 aus Beispiel 9 (Tabelle 4) wird destillativ aufgearbeitet. Dies geschieht in einer fraktionierten Destillation unter Verwendung einer Kolonne mit 8 theoretischen Trennstufen. Die Destillation erfolgt zunächst unter Normaldruck und anschließend unter vermindertem Druck. Die genauen Bedingungen und Zusammensetzungen der einzelnen Fraktionen lassen sich Tabelle 5 entnehmen.

Es lässt sich erkennen, dass vor allem Diacetonalkohl, Diacetonamin und Acetonin unter den Destillationsbedingungen nicht stabil sind. TAA zeigt unter den Destillationsbedingungen jedoch keine Tendenz zur Zersetzung. Die TAA-Ausbeute bei der Destillation beträgt 93.3% (Massenprozent) bei einer Reinheit von 94.9%.

| **Tabelle 5:** Destillative Aufreinigung des Triacetonamins; "%"-Angaben der Bestandteile in Massenprozent. | | | | | | | | | | | |
|---|---|---|---|---|---|---|---|---|---|---|---|
| **Probe** | **Menge** | **Wasser** | **Aceton** | **Mesityloxid** | **Diacetonalkohol** | **Diacetonamin** | **TMDH-Pyridin** | **Acetonin** | **TAA** | **Rest** | **Summe** |
| | **kg** | **%** | **%** | **%** | **%** | **%** | **%** | **%** | **%** | **%** | **%** |
| Einsatz | 28.00 | 2.10 | 2.89 | 5.38 | 7.00 | 3.41 | 1.97 | 3.23 | 61.00 | 13.02 | 100.00 |
| Fr.1 | 0.21 | 3.53 | 93.67 | 0 | 0 | 0 | 0 | 0.09 | 1.72 | 0.99 | 100.00 |
| Fr.2 | 0.99 | 17.54 | 42.17 | 28.08 | 0 | 3.05 | 0 | 0.59 | 0.02 | 8.55 | 100.00 |
| Fr. 3 untere Phase | 0.10 | 81.12 | 6.9 | 0.22 | 0.10 | 6.91 | 0.38 | 4.25 | 0.02 | 0.10 | 100.00 |
| Fr. 3 obere Phase | 1.40 | 5.01 | 5.22 | 77.56 | 0.26 | 0.99 | 5.28 | 0.2 | 0.02 | 5.46 | 100.00 |
| Fr.4 | 1.04 | 7.53 | 0.19 | 0.23 | 0.51 | 0.64 | 46.83 | 5.38 | 17.66 | 21.03 | 100.00 |
| Fr.5 | 16.80 | 0.08 | 0.03 | 0.01 | 0.00 | 0.00 | 0.02 | 0.03 | 94.87 | 4.97 | 100.00 |
| Rückstand | 3.04 | 4.14 | 0.47 | 0.07 | 0 | 0 | 0.13 | 0 | 4 | 91.19 | 100.00 |
| Kühlfalle | 3.50 | 1.5 | 2.55 | 0.20 | 0.03 | 0.06 | 0.12 | 0.02 | 0.22 | 95.30 | 100.00 |
| Einsatzmasse [kg] | 28.00 | 0.59 | 0.81 | 1.51 | 1.96 | 0.95 | 0.55 | 0.90 | 17.08 | 3.65 | |
| Masse in Destillat [kg] | 27.08 | 0.60 | 0.80 | 1.38 | 0.01 | 0.06 | 0.57 | 0.07 | 16.26 | 7.32 | |
| Fehler [%] | 3.29 | -2.36 | 0.66 | 8.63 | 99.48 | 93.74 | -3.77 | 91.82 | 4.82 | -100.85 | |

Bei der Destillation ist keine Bodensatzbildung festzustellen. Auch bei einer Wiederholung der Destillation ohne zwischenzeitliche Reinigung des Destillationssumpfes ist keine Bodensatzbildung und/oder die Bildung von Ablagerungen an Reaktoroberflächen oder Wärmetauscheroberflächen zu erkennen.

## Patentansprüche

1. Verfahren zur Herstellung von Triacetonamin, umfassend die Reaktionsschritte
a) Umsetzung eines Acetonäquivalents mit einer ersten Base, ausgewählt aus Ammoniak, in Gegenwart eines Katalysators, wobei ein Austragsgemisch erhalten wird, welches Aceton enthält;
b) Stoppen der katalytischen Aktivität des in Schritt a) eingesetzten Katalysators des Austragsgemisches aus Schritt a) durch Zugabe von mindestens 1 molaren Äquivalent, bezogen auf die Menge des in Schritt a) des Verfahrens eingesetzten Katalysators, einer weiteren Base, wobei ein Triacetonamin-Rohprodukt erhalten wird, welches Aceton enthält;
c) Entfernung von Aceton aus dem Triacetonamin-Rohprodukt aus Schritt b), wodurch ein acetonabgereichertes Triacetonamin-Rohprodukt erhalten wird, wobei im acetonabgereicherten Triacetonamin-Rohprodukt ein Acetongehalt von unter 40 Massenprozent vorliegt;
d) Extraktion des acetonabgereicherten Triacetonamin-Rohprodukts aus Schritt c) durch Zusatz einer Base, wodurch die nach Schritt c) des Verfahrens erhaltene Lösung entsalzt wird, und wobei ein extrahiertes Triacetonamin-Rohprodukt erhalten wird.

2. Verfahren gemäß Anspruch 1, **dadurch gekennzeichnet, dass** in einem weiteren Schritt e) das extrahierte Triacetonamin-Rohprodukt aus Schritt d) zu Triacetonamin gereinigt wird.

3. Verfahren gemäß Anspruch 1 oder 2, **dadurch gekennzeichnet, dass** das Acetonäquivalent eine oder mehrere der Verbindungen ausgewählt aus der Gruppe bestehend aus Aceton, Mesityloxid, Diacetonalkohol, Phoron, Diacetonamin, Acetonin ist.

4. Verfahren gemäß einem oder mehreren der Ansprüche 1 bis 3, **dadurch gekennzeichnet, dass** die weitere Base in Schritt b) ausgewählt ist aus der Gruppe bestehend aus einem Alkalimetallhydroxid, einem Erdalkalimetallhydroxid, einem Alkalimetallcarbonat, einem Erdalkalimetallcarbonat.

5. Verfahren gemäß einem oder mehreren der Ansprüche 1 bis 4, **dadurch gekennzeichnet, dass** die Menge an in Schritt b) zugegebener weiterer Base im Bereich von 1.0 bis 2.0 molaren Äquivalenten, bezogen auf die Menge des in Schritt a) eingesetzten Katalysators, liegt.

6. Verfahren gemäß einem oder mehreren der Ansprüche 1 bis 5, **dadurch gekennzeichnet, dass** zusätzlich zwischen Schritt b) und Schritt c) eine Phasentrennung erfolgt.

7. Verfahren gemäß einem oder mehreren der Ansprüche 1 bis 6, **dadurch gekennzeichnet, dass** die Umsetzung in Schritt a) bei Temperaturen im Bereich von 40 - 80°C durchgeführt wird.

8. Verfahren gemäß einem oder mehreren der Ansprüche 1 bis 7, **dadurch gekennzeichnet, dass** die Entfernung von Aceton aus dem Triacetonamin-Rohprodukt in Schritt c) destillativ erfolgt.

9. Verfahren gemäß einem oder mehreren der Ansprüche 1 bis 8, **dadurch gekennzeichnet, dass** der Katalysator in Schritt a) ausgewählt ist aus der Gruppe bestehend aus Brönsted-Säuren, Ammoniumsalzen einer Brönstedtsäure, Phosphoniumsalzen einer Brönstedtsäure, Lewissäuren.

10. Verfahren gemäß einem oder mehreren der Ansprüche 1 bis 9, **dadurch gekennzeichnet, dass** der Katalysator in Schritt a) Ammoniumchlorid oder Ammoniumnitrat ist.

11. Verfahren gemäß einem oder mehreren der Ansprüche 1 bis 10, **dadurch gekennzeichnet, dass** das Molverhältnis von Ammoniak zu Katalysator in Schritt a) im Bereich 1 : 0.8 bis 1 : 0.02 liegt.

12. Verfahren gemäß einem oder mehreren der Ansprüche 1 bis 11, **dadurch gekennzeichnet, dass** die Extraktion des acetonabgereicherten Triacetonamin-Rohprodukts in Schritt d) als Kreuzstromextraktion oder als Gegenstromextraktion durchgeführt wird.

13. Verfahren gemäß einem oder mehreren der Ansprüche 2 bis 12, **dadurch gekennzeichnet, dass** das extrahierte Triacetonamin-Rohprodukt in Schritt e) destillativ zu Triacetonamin gereinigt wird.

## Claims

1. Process for the preparation of triacetone amine, comprising the reaction steps
a) reaction of an acetone equivalent with a first base, selected from ammonia, in the presence of a catalyst, giving a product mixture which comprises acetone;
b) stopping the catalytic activity of the catalyst used in step a) of the product mixture from step a) by adding at least 1 molar equivalent, based on the amount of catalyst used in step a) of the process, of a further base, giving a triacetone amine crude product which comprises acetone;
c) removal of acetone from the triacetone amine crude product from step b), giving an acetone-depleted triacetone amine crude product, an acetone content of below 40 percent by mass being present in the acetone-depleted triacetone amine crude product;
d) extraction of the acetone-depleted triacetone amine crude product from step c) by adding a base, whereby the solution obtained according to step c) of the process is desalted, giving an extracted triacetone amine crude product.

2. Process according to Claim 1, **characterized in that**, in a further step e), the extracted triacetone amine crude product from step d) is purified to give triacetone amine.

3. Process according to Claim 1 or 2, **characterized in that** the acetone equivalent is one or more of the compounds selected from the group consisting of acetone, mesityl oxide, diacetone alcohol, phorone, diacetone amine, acetonin.

4. Process according to one or more of Claims 1 to 3, **characterized in that** the further base in step b) is selected from the group consisting of an alkali metal hydroxide, an alkaline earth metal hydroxide, an alkali metal carbonate, an alkaline earth metal carbonate.

5. Process according to one or more of Claims 1 to 4, **characterized in that** the amount of further base added in step b) is in the range from 1.0 to 2.0 molar equivalents, based on the amount of catalyst used in step a).

6. Process according to one or more of Claims 1 to 5, **characterized in that** a phase separation additionally takes place between step b) and step c).

7. Process according to one or more of Claims 1 to 6, **characterized in that** the reaction in step a) is carried out at temperatures in the range from 40 - 80°C.

8. Process according to one or more of Claims 1 to 7, **characterized in that** the removal of acetone from the triacetone amine crude product in step c) takes place by distillation.

9. Process according to one or more of Claims 1 to 8, **characterized in that** the catalyst in step a) is selected from the group consisting of Brönsted acids, ammonium salts of a Brönsted acid, phosphonium salts of a Brönsted acid, Lewis acids.

10. Process according to one or more of Claims 1 to 9, **characterized in that** the catalyst in step a) is ammonium chloride or ammonium nitrate.

11. Process according to one or more of Claims 1 to 10, **characterized in that** the molar ratio of ammonia to catalyst in step a) is in the range 1 : 0.8 to 1 : 0.02.

12. Process according to one or more of Claims 1 to 11, **characterized in that** the extraction of the acetone-depleted triacetone amine crude product in step d) is carried out as a crossflow extraction or as a countercurrent extraction.

13. Process according to one or more of Claims 2 to 12, **characterized in that** the extracted triacetone amine crude product in step e) is purified by distillation to give triacetone amine.

## Revendications

1. Procédé de fabrication de triacétonamine, comprenant les étapes de réaction suivantes :
a) la mise en réaction d'un équivalent d'acétone avec une première base, choisie parmi l'ammoniac, en présence d'un catalyseur, un mélange de sortie étant obtenu, qui contient de l'acétone ;
b) l'arrêt de l'activité catalytique du catalyseur utilisé à l'étape a) dans le mélange de sortie de l'étape a) par ajout d'au moins 1 équivalent molaire, par rapport à la quantité de catalyseur utilisé à l'étape a) du procédé, d'une autre base, un produit brut de triacétonamine étant obtenu, qui contient de l'acétone ;
c) l'élimination de l'acétone du produit brut de triacétonamine de l'étape b), un produit brut de triacétonamine appauvri en acétone étant obtenu, une teneur en acétone de moins de 40 pour cent en masse étant présente dans le produit brut de triacétonamine appauvri en acétone ;
d) l'extraction du produit brut de triacétonamine appauvri en acétone de l'étape c) par ajout d'une base, la solution obtenue après l'étape c) du procédé étant ainsi dessalée, et un produit brut de triacétonamine extrait étant obtenu.

2. Procédé selon la revendication 1, **caractérisé en ce que**, lors d'une étape e) supplémentaire, le produit brut de triacétonamine extrait de l'étape d) est purifié en triacétonamine.

3. Procédé selon la revendication 1 ou 2, **caractérisé en ce que** l'équivalent d'acétone est un ou plusieurs des composés choisis dans le groupe constitué par l'acétone, l'oxyde de mésityle, l'alcool diacétonique, la phorone, la diacétonamine, l'acétonine.

4. Procédé selon une ou plusieurs des revendications 1 à 3, **caractérisé en ce que** l'autre base à l'étape b) est choisie dans le groupe constitué par un hydroxyde de métal alcalin, un hydroxyde de métal alcalino-terreux, un carbonate de métal alcalin, un carbonate de métal alcalino-terreux.

5. Procédé selon une ou plusieurs des revendications 1 à 4, **caractérisé en ce que** la quantité de l'autre base ajoutée à l'étape b) se situe dans la plage allant de 1,0 à 2,0 équivalents molaires, par rapport à la quantité du catalyseur utilisé à l'étape a).

6. Procédé selon une ou plusieurs des revendications 1 à 5, **caractérisé en ce qu'**une séparation de phases a en outre lieu entre l'étape b) et l'étape c).

7. Procédé selon une ou plusieurs des revendications 1 à 6, **caractérisé en ce que** la réaction à l'étape a) est réalisée à des températures dans la plage allant de 40 à 80 °C.

8. Procédé selon une ou plusieurs des revendications 1 à 7, **caractérisé en ce que** l'élimination de l'acétone du produit brut de triacétonamine à l'étape c) a lieu par distillation.

9. Procédé selon une ou plusieurs des revendications 1 à 8, **caractérisé en ce que** le catalyseur à l'étape a) est choisi dans le groupe constitué par les acides de Bronsted, les sels d'ammonium d'un acide de Bronsted, les sels de phosphonium d'un acide de Bronsted, les acides de Lewis.

10. Procédé selon une ou plusieurs des revendications 1 à 9, **caractérisé en ce que** le catalyseur à l'étape a) est le chlorure d'ammmonium ou le nitrate d'ammonium.

11. Procédé selon une ou plusieurs des revendications 1 à 10, **caractérisé en ce que** le rapport molaire entre l'ammoniac et le catalyseur à l'étape a) se situe dans la plage allant de 1:0,8 à 1:0,02.

12. Procédé selon une ou plusieurs des revendications 1 à 11, **caractérisé en ce que** l'extraction du produit brut de triacétonamine appauvri en acétone à l'étape d) est réalisée sous la forme d'une extraction à courants croisés ou d'une extraction à contre-courant.

13. Procédé selon une ou plusieurs des revendications 2 à 12, **caractérisé en ce que** le produit brut de triacétonamine extrait est purifié par distillation en triacétonamine à l'étape e).
